# EUROPÄISCHE PATENTSCHRIFT

(11) **EP 1 307 210 B1**
(45) Veröffentlichungstag und Bekanntmachung des Hinweises auf die Patenterteilung: **07.06.2006**
(21) Anmeldenummer: 01955372.6
(22) Anmeldetag: 26.07.2001
(51) Int. Cl.: A61K 35/00, C07J 75/00

(54) **VERFAHREN UND VORRICHTUNG ZUR ANREICHERUNG UND STABILISIERUNG VON KONJUGIERTEN OESTROGENEN AUS STUTENHARN**
METHOD AND DEVICE FOR CONCENTRATING AND STABILISING CONJUGATED OESTROGENS FROM THE URINE OF MARES
PROCEDE ET DISPOSITIF DE CONCENTRATION ET DE STABILISATION D'OESTROGENES CONJUGUES ISSUS DE L'URINE D'UNE JUMENT

(30) Priorität: 01.08.2000 DE 10037389
(43) Veröffentlichungstag der Anmeldung: 07.05.2003
(73) Patentinhaber: Solvay Pharmaceuticals GmbH, 30173 Hannover (DE)
(72) Erfinder: AHNSORGE, Jürgen, 31535 Neustadt am Rübenberge (DE); BAN, Ivan, 30539 Hannover (DE); RASCHE, Heinz-Helmer, 31303 Burgdorf (DE)
(74) Vertreter: Gosmann, Martin
(86) Internationale Anmeldenummer: PCT/EP2001/008657
(87) Internationale Veröffentlichungsnummer: WO 2002/009732

(56) Entgegenhaltungen:
- WO-A-98/08525
- US-A- 2 834 712

## Beschreibung

Die vorliegende Erfindung betrifft ein Verfahren und eine Vorrichtung zur Anreicherung und Stabilisierung von konjugierten Oestrogenen aus dem Harn trächtiger Stuten an festen Trägern, um geeignetes Ausgangsmaterial für die Herstellung von Pharmazeutika zu gewinnen, die das natürliche Gemisch dieser konjugierten Oestrogene als Wirkkomponente enthalten.

Oestrogene werden in der Medizin zur Hormonsubstitutionstherapie eingesetzt. Insbesondere werden Oestrogengemische zur Behandlung und Prophylaxe der bei Frauen auftretenden Beschwerden der Wechseljahre nach natürlicher oder artifizieller Menopause eingesetzt. Hierbei haben sich natürliche Gemische konjugierter Oestrogene, wie sie im Harn trächtiger Stuten vorliegen, als besonders wirksam und gut verträglich erwiesen.

Der gelöste Feststoffgehalt im Harn trächtiger Stuten (= pregnant mares' urine, im folgenden abgekürzt als "PMU") kann natürlicherweise in weiten Bereichen schwanken und im allgemeinen in einem Bereich von 40 - 90 g Trockensubstanz pro Liter liegen. Neben Harnstoff und sonstigen üblichen Harninhaltsstoffen sind in dem Feststoffgehalt des PMU phenolische Bestandteile in Mengen von ca. 2 - 5 Gew.-% bezogen auf Trockensubstanz enthalten. Unter diesen phenolischen Bestandteilen befinden sich Kresole und das als HPMF bekannte Dihydro-3,4-bis[(3-hydroxyphenyl)methyl)-2(3H)-furanon. Diese können in freier oder konjugierter Form vorliegen. In dem PMU ist ein natürliches Gemisch von Oestrogenen enthalten, welches weitgehend in konjugierter Form, z.B. als Schwefelsäurehalbester-Natriumsalz (im folgenden abgekürzt als "Sulfatsalz"), vorliegt. Der Gehalt an konjugierten Oestrogenen (= conjugated estrogen, im folgenden abgekürzt als "CE") kann berechnet als Oestrogensulfatsalz und bezogen auf Trockensubstanz zwischen 0,3 und 1 Gew.-% betragen.

Zur direkten Aufbereitung und Gewinnung der im PMU enthaltenen konjugierten Oestrogene sind im Stand der Technik verschiedene Verfahrensweisen beschrieben. Üblicherweise werden konjugierte Oestrogene enthaltende Extrakte aus dem PMU durch Extraktion mit einem polaren mit Wasser nicht oder nur wenig mischbaren organischen Lösemittel wie beispielsweise Essigsäureethylester, n-Butanol oder Cyclohexanol gewonnen. Bei derartigen Flüssig-Flüssig-Extraktionen treten jedoch eine Vielzahl von Problemen, wie starke Schaumbildung, Sedimentbildung, Emulsionsbildung und schlechte Phasentrennung auf. Es werden im allgemeinen mehrere Extraktionsschritte benötigt, was zu Verlusten und einer nur teilweisen Gewinnung des Oestrogengehaltes führt. Zur Vermeidung dieser Nachteile wurden im Stand der Technik daher eine Reihe von Festphasen-Extraktionsverfahren vorgeschlagen.

Für die Aufbereitung von kleinen Mengen an Harn- und Plasmaflüssigkeiten für eine analytische Bestimmung von Oestrogenen mittels Gaschromatographie beschreiben Heikkinnen et al. (Clin. Chem. 27/7,(1981), 1186 - 1189) und Shackleton et al. (Clinica Chimica Acta 107(1980) 231 - 243) eine Festphasen-Extraktion von Oestrogenen mittels einer Kartusche mit Octadecylsilanreste enthaltendem silanisiertem Kieselgel (Sep-Pak^{R} C¹⁸-cartridge, Hersteller Waters Ass. Inc. Milford, MA, USA). Hierbei werden die Oestrogene mit Methanol von der Kartusche eluiert.

Von H.L. Bradlow wurde 1968 (siehe Steroids 11 (1968), 265 - 272) vorgeschlagen, zur Extraktion von konjugierten Oestrogenen aus Harn Amberlite XAD-2^{R}, ein neutrales unpolares hydrophobes Polystyrolharz der Fa. Rohm und Haas, einzusetzen. Die angegebene Adsorptionskapazität ist gering. Nach Bradlow wird ein gegebenenfalls verdünnter Harn mit einer niedrigen Durchflußgeschwindigkeit durch eine das Harz enthaltende Säule geleitet. Die Oestrogene werden mit Methanol oder Ethanol eluiert.

Neuere Patentanmeldungen beschreiben Verfahren zur Gewinnung eines das natürliche Gemische konjugierter Oestrogene aus Stutenharn enthaltenden Extraktes durch Festphasenextraktion des Gemisches konjugierter Oestrogene aus dem Harn trächtiger Stuten z.B. an RP-Kieselgel (WO 98/08525) oder an nichtionischen semipolaren polymeren Adsorberharzen (WO 98/08526).

Neben der vorstehend beschriebenen Optimierung der unmittelbaren, vollständigen Aufarbeitung von Harn trächtiger Stuten (PMU) zur Gewinnung natürlicher Gemische konjugierter Oestrogene (CE) sind auch die der Aufarbeitung vorgelagerten Schritte, wie z.B. Sicherstellung einer Oestrogen-schonenden Lagerung und Handhabung des gesammelten Harns am Sammelort von besonderer Bedeutung für die Ausbeute und Qualität des oestrogenhaltigen Rohmaterials und des daraus zu isolierenden natürlichen Gemisches konjugierter Oestrogene. In diesem Zusammenhang ist auch eine Optimierung des Transports des angesammelten oestrogenhaltigen Rohmaterials vom Sammelort zum Ort der eigentlichen Aufarbeitung und Isolierung der konjugierten Oestrogene anzustreben.

Eine vollständige Aufbereitung von PMU z.B. durch Festphasenextraktion erfordert chemisch-pharmazeutisch qualifiziertes Fachpersonal, um unter gut steuerbaren Bedingungen die hohen Anforderungen an Reinheit und Qualität einer als pharmazeutischer Wirkstoff zu verwendenden Substanz oder Substanzgemisches einhalten zu können. Das Sammeln des Harns (PMU) findet jedoch üblicherweise im normalen Lebensumfeld der trächtigen Stuten statt, d.h. in Gestüten, einer bäuerlichen Umgebung und oft auch an entlegeneren Orten. In der Regel liegt hier nur eine einfache Infrastruktur vor und das Sammeln des Harns (PMU) und dessen Handhabung erfolgt gewöhnlich durch einfaches, angelerntes Personal, an das in bezug auf die Handhabung des gesammelten Harns keine zu hohen Anforderungen gestellt werden können.

Konjugierte Oestrogene (CE), in der Zusammensetzung wie im Harn trächtiger Stuten ausgeschieden, sind aber ein komplexes Gemisch, welches insbesondere Natriumestronsulfat, Natriumequilinsulfat sowie weitere CE enthält. Für die Isolierung der CE ist es sehr wichtig, daß der PMU möglichst schnell in frischem Zustand aufgearbeitet wird. Bei längerer Lagerung beginnt der Harn sich rasch zu zersetzen. Der Harn färbt sich dunkel, riecht nach Ammoniak und der Gehalt an CE nimmt sehr stark ab. Die Zersetzungsgeschwindigkeit hängt von den Lagerungsbedingungen und von Reinheitskriterien ab. Der gesammelte Harn (PMU), wobei es sich um große Flüssigkeitsmengen handelt, muß daher bisher täglich vom Sammelort zu einer zentralen Aufarbeitungsstelle transportiert werden, um eine möglichst rasche Aufarbeitung zu gewährleisten.

Es besteht daher ein dringender Bedarf an geeigneten Verfahren und Vorrichtungen zur möglichst produktschonenden und einfach handhabbaren Anreicherung und Stabilisierung von im Harn trächtiger Stuten enthaltenen konjugierten Oestrogenen (CE) direkt am Sammelort des Harns (PMU), sowie zum möglichst effizienten Transport vom Sammelort zum Ort der Aufarbeitung und Isolierung.

Aufgabe der vorliegenden Erfindung ist es somit, unter Vermeidung der vorstehend beschrieben Nachteile, ein für die Gewinnung des natürlichen Gemisches konjugierter Oestrogene geeignetes technisches Verfahren und eine geeignete Vorrichtung zur Anreicherung und Stabilisierung von konjugierten Oestrogenen (CE) aus dem gesammelten Harn trächtiger Stuten (PMU) zu entwickeln. Insbesondere sollen das Verfahren und die Vorrichtung eine produktschonende einfach handhabbare Anreicherung und Stabilisierung der konjugierten Oestrogene aus dem PMU am Sammelort des Harns sowie einen möglichst effizienten Transport des derart bereitgestellten oestrogenhaltigen Rohmaterials vom Sammelort zum Ort der eigentlichen Aufarbeitung und Isolierung des natürlichen Gemisches konjugierter Oestrogene ermöglichen.

Es wurde nun überraschenderweise gefunden, daß konjugierte Oestrogene (CE) an festen Trägern (= Adsorbenzien) gebunden sehr stabil und über längere Zeiten ohne irgendeinen Hinweis auf Zersetzung haltbar sind.

Die vorliegende Erfindung betrifft daher ein Verfahren zur praxisgerechten, dezentralen, d.h. insbesondere aufstallungs- und/oder weidenahen Anreicherung und Stabilisierung von Gemischen konjugierter Oestrogene (CE) aus dem Harn trächtiger Stuten (PMU), welches sich dadurch auszeichnet, daß
- das im Harn trächtiger Stuten (PMU)enthaltene natürliche Gemisch konjugierter Oestrogene (CE) an einem festen Träger (= Adsorbens) angereichert und stabilisiert wird, indem
- eine vorgegebene maximale Gesamtmenge einer gesammelten, gegebenenfalls zuvor gesiebten Harnflüssigkeit aus einem Vorratsgefäß kontinuierlich oder in diskreten Teilmengen mit vorgegebener Strömungsgeschwindigkeit durch eine aufrecht angeordnete Kartusche gepumpt wird und die abfließende Harnflüssigkeit verworfen wird, wobei
- die Kartusche mit einem (ständig) von Flüssigkeit umgebenen, zur Adsorption einer vordefinierten Menge des in der Harnflüssigkeit enthaltenen Gemisches konjugierter Oestrogene (CE) geeigneten Adsorbens gefüllt ist, und wobei
- die als Endpunktkontrolle für die Beladung des Adsorbens vorgegebene, maximale Gesamtmenge der durch die Kartusche gepumpten Harnflüssigkeit auf die maximale Beladungskapazität des Adsorbens für die in der Harnflüssigkeit enthaltenen konjugierten Oestrogene (CE) abgestimmt ist.

Der Begriff "praxisgerecht" bedeutet hierbei, daß das Verfahren an Bedingungen der Sammelpraxis vor Ort angepaßt, also einfach und sicher durch angelerntes Personal handhabbar ist; d.h. es ist unkompliziert und unter weitgehendem Ausschluß möglicher Fehlbedienungen durchführbar.

Der Begriff "dezentral" bedeutet hierbei, daß das Verfahren am jeweiligen Ort des Harn-Sammelbetriebes ohne spezielle chemisch-pharmazeutische Einrichtungen, d.h., insbesondere aufstallungs- und/oder weidenah ausgeführt werden kann. Als "Kartusche" wird hierbei jede Art geschlossener Säule mit schließbarem Zu- und Ablauf sowie Anschlußelementen, z.B. Schnellkupplungen, verstanden. Der Begriff "Kartusche" umfaßt somit im Rahmen der vorliegenden Erfindung auch Säulen, wie diese üblicherweise z.B. im Labor, im Technikum bzw. im chemischen Betrieb Verwendung finden, und die wie vorstehend genannt für den erfindungsgemäßen Einsatz ausgerüstet sind.

Nach dem erfindungsgemäßen Verfahren wird in vorteilhafter Weise ein für die Herstellung von Pharmazeutika, die das natürliche Gemisch konjugierter Oestrogene aus dem PMU als Wirkkomponente enthalten, dienendes Roh- bzw. Ausgangsmaterial bereitgestellt, in dem der natürliche Oestrogen-Gemisch-Gehalt des PMU praktisch vollständig am Adsorbens (Träger) angereichert und über längere Zeit unter den normalen Umgebungsbedingungen des jeweiligen Sammelortes stabilisiert werden kann. Nach vollständiger (maximaler) Beladung kann die Kartusche leicht ausgetauscht und durch eine Kartusche mit unbeladenem Adsorbens ersetzt werden. Die Kartuschen mit beladenem Adsorbens können noch geraume Zeit vor Ort bei Umgebungstemperatur oder gewünschtenfalls auch unter Kühlung bis herab zu etwa 4 °C und unter bestimmten Voraussetzungen auch tiefgekühlt bis herab zu etwa - 20 °C gelagert werden, z.B. bis eine gewisse Anzahl beladener Kartuschen für einen zweckmäßigen Sammeltransport zur zentralen Aufarbeitungsstelle vorliegt. Der Transport kann unter Umgebungstemperaturen oder wie vorstehend für die Lagerung beschrieben unter Kühlung bzw. Tiefkühlung erfolgen. Die eigentliche Aufarbeitung und Isolierung des natürlichen Gemisches der konjugierten Oestrogene (CE) kann dann in der zentralen Aufarbeitungsstelle in einer für das jeweilige Adsorbens üblichen weise, z.B. durch Waschen und Elution der CE vom Adsorbens und Weiterverarbeitung des Eluats in gewohnter Weise erfolgen. Beispielsweise kann das Eluat auf an sich bekannte Weise weiter eingeengt werden, um ein weitgehend oder vollständig von organischem Lösemittel befreites, zur galenischen Weiterverarbeitung geeignetes Konzentrat zu erhalten. Gewünschtenfalls kann auch durch andere geeignete Trocknungsverfahren, wie z.B. durch Sprühtrocknung, durch Wirbelschichttrocknung, durch Gefriertrocknung oder Vakuumtrocknung, ein elutionsmittelfreies Feststoffgemisch hergestellt werden. Sowohl das Oestrogengemisch enthaltende Eluat als auch ein daraus hergestelltes Konzentrat oder sprühgetrocknetes Feststoffprodukt können in an sich bekannter Weise in feste oder flüssige galenische Zubereitungen wie beispielsweise Tabletten, Dragees, Kapseln oder Emulsionen eingearbeitet werden.

Neben dem erfindungsgemäßen Verfahren betrifft die Erfindung auch eine in diesem Verfahren einsetzbare Vorrichtung, die den einfachen Bedingungen am Einsatzort in zweckmäßiger Weise Rechnung trägt und eine sichere, produktschonende Durchführung des Verfahrens gewährleistet. Diese erfindungsgemäße Vorrichtung eignet sich zur praxisgerechten, dezentralen, d.h. insbesondere aufstallungs- oder weidenahen, Anreicherung und Stabilisierung. Im folgenden werden noch einige weitere für die Verfahrensdurchführung wichtige Verfahrensparameter allgemein erläutert. In bezug auf spezielle konstruktive bzw. apparative Ausgestaltungen von Vorrichtungen, die sich zur Durchführung des erfindungsgemäßen Verfahrens eignen, wird auf die detaillierte Beschreibung der erfindungsgemäßen Vorrichtung weiter unten verwiesen, die gegebenenfalls ergänzend zur nachfolgenden allgemeinen Beschreibung der Verfahrensdurchführung heranzuziehen ist.

Für das erfindungsgemäße Verfahren kann der gesammelte PMU als solcher eingesetzt werden und die täglich gesammelte Menge PMU wird hierfür zunächst in einen Vorratsbehälter vorgehalten. Gegebenenfalls empfiehlt sich die vorherige Abtrennung von groben mechanischen Verunreinigungen wie Stroh, Heu oder dergleichen, z.B. mittels eines einfachen grobmaschigen Siebes beim Befüllen des Vorratsbehälters mit dem frisch gesammelten PMU. Gewünschtenfalls können zur Verminderung der Keim- und Virenzahl dem im Vorratsbehälter gesammelten Harn Konservierungsmittel, Germizide, Bakterizide und/oder Anthelmintika zugesetzt werden.

Aus dem Vorratsbehälter wird der PMU regelmäßig in Abhängigkeit von der angefallenen Menge an Harn, z.B. ein- bis zweimal täglich, mittels einer Pumpe, z.B. mittels einer Schlauchpumpe, zur Adsorbens enthaltenden Kartusche gepumpt. Diese Kartusche weist üblicherweise Abmessungen und Gewichte auf, die ein manuelles Hantieren durch normal kräftige Personen erlauben. In bezug auf das Verfahren empfiehlt es sich, die Innenabmessungen der Kartusche für das aufzunehmende Adsorbensbett so zu bemessen, daß sie in etwa 30 bis 50 1 Adsorbens aufnehmen kann und der PMU vom Einlaß bis zum Auslaß etwa eine Wegstrecke von 80 bis 120 cm, vorzugsweise zurücklegen muß. Erfindungsgemäß erfolgt die Adsorption der konjugierten Oestrogene an das Adsorbens durch Rontaktierung des PMU mit dem Adsorbens, indem die Harnflüssigkeit in eine das Adsorbens enthaltende aufrecht angeordnete Kartusche, wahlweise z.B. kopf- oder fußseitig, eingeführt und darin während des Durchflusses für eine zur Adsorption des Oestrogengehaltes ausreichende Zeit mit dem Adsorbens in Kontakt gehalten wird, bis der Restharn schließlich an der entgegengesetzten Seite die Kartusche verläßt. Der PMU wird hierbei so, z.B. kopf- oder fußseitig, in die Kartusche eingeführt, daß sichergestellt ist, daß das Adsorbens ständig von Flüssigkeit umgeben ist, um ein unerwünschtes Trockenlaufen der Kartusche möglichst auszuschließen.

Die Kontaktzeit bzw. die Strömungsgeschwindigkeit ist auf das jeweilige Adsorbens abgestimmt und sollte bei für das Verfahren geeignete Adsorbenzien (= Beladegeschwindigkeit) im Bereich von 3 bis 10 Adsorber-Bettvolumen/h, vorzugsweise 4 bis 6 Adsorber-Bettvolumen/h liegen. Beispielhaft geeignete Beladegeschwindigkeiten sind z.B. 4,5 bis 5,5 Adsorber-Bettvolumen/h.

Spezielle Kontaktzeiten und Strömungsgeschwindigkeiten sind für besonders bevorzugte Adsorbenzien weiter unten bei der Beschreibung dieser Adsorbenzien angegeben.

Die maximale Gesamtmenge an Harnflüssigkeit, die über die Kartusche geleitet werden kann hängt von der Adsorptionskapazität des jeweiligen Adsorbens ab und sollte bei für das Verfahren geeigneten Adsorbenzien im Bereich von 20 bis 60 Adsorber-Bettvolumen, vorzugsweise von 30 bis 40 Adsorber-Bettvolumen, liegen. Üblicherweise dürfte die maximale Gesamtmenge an Harnflüssigkeit, die zur Beladung über die Kartusche gleitet werden kann, je nach Adsorbens und Kartuschengröße daher im Bereich von 900 bis 2.000 1 liegen. Der jeweilige Endwert der Durchflußmenge wird dem Bedienungspersonal am Sammelort für den jeweiligen Kartuschentyp fest vorgegeben. Zur Messung der über die Kartusche gepumpten Menge an Harnflüssigkeit kann daher ein einfacher Durchflußmesser, vorzugsweise z.B. eine Wasseruhr, eingesetzt werden. Nach Erreichen der maximalen Beladung kann gegebenenfalls auf eine parallele zweite Kartusche umgeschaltet und der Adsorptionsvorgang an dieser fortgesetzt werden.

Ist der für die Kartusche vorgegebene Endwert erreicht, wird die Kartusche mit den adsorbierten CE gewünschtenfalls noch z.B. mit Wasser und/oder einer anderen geeigneten wäßrigen Waschlösung gewaschen, bevor die Flüssigkeitszufuhr unterbrochen wird. Danach werden der Flüssigkeitsablauf und der Flüssigkeitszulauf geschlossen und dann die Verbindung, z.B. eine einfache Schnellkupplung, des Flüssigkeitszulaufs zur Kartusche gelöst. Die beladene Kartusche kann danach entnommen und an einem geeigneten Ort bis zum Abtransport zur zentralen Aufarbeitungsstelle gelagert und eine frische, unbeladene Kartusche an den Flüssigkeitszulauf angeschlossen werden.

Die Kartusche kann nach der Beladung mit PMU, z.B. vor dem Austauschen einer vollständig mit PMU beladenen Kartusche oder auch vor Erreichen der Gesamtbeladungskapazität nach beliebigen einzelnen Teilbeladungsschritten, gewünschtenfalls noch mit Wasser und/oder einer anderen geeigneten wäßrigen Waschlösung, z.B. einer basischen Waschlösung wie insbesondere z.B. verdünnte wäßrige Natronlauge (z.B. wäßrige 0,5-2 N NaOH) gespült werden, um Rest-Harnflüssigkeit aus der Kartusche zu entfernen. Die Menge an Waschlösung ist nicht kritisch und wird vorzugsweise so gewählt, daß sie ausreicht, um den Restharn aus der Kartusche zu verdrängen, ohne daß dabei nennenswerte Mengen konjugierter Oestrogene mit ausgewaschen werden. Als zweckmäßig erweist sich beispielsweise die Verwendung von 1 - 3, vorzugsweise etwa 2 Bettvolumen Waschflüssigkeit pro Bettvolumen Adsorbens. Hierbei wird das Waschwasser oder die Waschlösung zweckmäßigerweise durch die das Adsorbens enthaltende Kartusche mit einer Durchflußgeschwindigkeit von 3 bis 10, vorzugsweise 5 bis 7, Vol.-Teilen Waschwasser pro 1 Vol.-Teil Adsorbens/Stunde geleitet. Das in der Kartusche mit CE beladene Adsorbens kann als Roh- bzw. Ausgangsmaterial für die Isolierung von reinem CE für die Herstellung von das natürliche Gemisch konjugierter Oestrogene enthaltenden Arzneimitteln dienen.

In einer Variante der Erfindung kann die Kartusche nach dem Waschen mit Wasser und/oder Waschlösung zur weiteren Stabilisierung der adsorbierten CE abschließend noch mit einer konservierenden Lösung, z.B. einer Konservierungsmitel enthaltenden Lösung, einer pH-eingestellten wäßrigen Lösung und/oder einer wäßrigen Salzlösung gespült werden, bevor die Kartusche aus der Vorrichtung gelöst und durch eine frische ersetzt wird. Zur Verhinderung der Verkeimung der Säule, insbesondere bei Lagerung oder Transport ohne Kühlung können übliche Konservierungsmittel und z.B. auch Germizide, Bakterizide und/oder Anthelmintika eingesetzt werden. Eine pH-Einstellung des Waschwassers wird sich nach dem jeweils verwendeten Adsorbens richten. Als Salzlösungen können unterschiedlich konzentrierte wäßrige Lösungen anorganischer Salze verwendet werden, für die Beispiele in Tabelle III.1 angegeben sind. Der Einsatz von Salzlösungen empfiehlt sich insbesondere auch dann, wenn die mit CE beladenen Kartuschen gekühlt bzw. insbesondere tiefgekühlt gelagert werden sollen. Bevorzugte Salzlösungen sind Kochsalzlösungen mit Salzkonzentrationen von etwa 10 bis 35 Gew.-%, vorzugsweise 25 bis 33 Gew.-%.

In einer bevorzugten Ausgestaltung des erfindungsgemäßen Verfahrens wird der gesammelte Harn, bevor er aus dem Vorratsbehälter zur Kartusche gepumpt wird, vorzugsweise von Schleim- und feinteiligeren Feststoffen befreit. Es ist daher zweckmäßig, wenn man die Harnflüssigkeit aus dem Vorratsbehälter zunächst erst über einen oder mehrere Vorfilter leitet, bevor die Harnflüssigkeit über die Kartusche gepumpt wird. So kann der PMU beispielsweise über wenigstens eine an sich bekannte Trenneinrichtung, z.B. über eine Filtrationsanlage mit wenigstens einen Tiefenfilter oder Anschwemmfilter geleitet werden. Als Trenneinrichtung kann z.B. ein Tiefenfilter mit Sandbett oder eine handelsübliche Filterkartusche dienen, oder es können handelsübliche Anschwemmfilter oder Plattenfilter, Kerzenfilter, Filterbeutel oder auch Filtrationsrohre eingesetzt werden. Die Filter können einzeln oder mehrere auch in beliebiger Kombination miteinander, z.B. in Serie hintereinander und/oder parallel geschaltet eingesetzt werden. Gewünschtenfalls können der Harnflüssigkeit (PMU) vor der Filtration noch Filtrationshilfsmittel zugesetzt werden. Geeignete Filtrationshilfsmittel sind z.B. solche, die Calciumcarbonat und/oder Muccine binden und somit die Harnfiltration verbessern. Gegebenenfalls kann dem Filter noch ein Partikelabscheider nachgeschaltet sein, z.B. im Falle eines Sandbetts als Filter ein nachgeschalteter Sandabscheider.

Dem Fachmann sind die Prinzipien bzw. die Ausführungsweise von Filtration an sich geläufig. Im Hinblick auf die durch die Erfindung zu lösende Aufgabe kommen daher die üblichen Prinzipien der Klärfiltration für den erfindungsgemäßen Einsatz in Betracht. Die Klärfiltration zielt auf die Reinigung der flüssigen Phase ab, so daß für sie die Reinheit des Filtrates wesentliche Bewertungsgröße für das Erreichen des Prozeßzieles ist. Der Trennprozeß wird grundsätzlich durch das Zusammenwirken zwischen dem Filtermittel und der Suspension realisiert. Das ist sowohl bei der Auswahl der Filtermittel, der Prozeßmodellierung als auch bei der verfahrenstechnischen Auslegung von Filterapparaten zu beachten. In den meisten Fällen beeinflußt der abgeschiedene Feststoff den Prozeßverlauf maßgeblich. So übernimmt bei hinreichendem Anteil an Feststoff und bei dessen Fähigkeit zur Brückenbildung (die Teilchen stützen sich über dem Filtermittel ab, ohne es zu verstopfen) der sich bildende und fortlaufend anwachsende Filterkuchen die Funktion des Filtermittels. In diesem Fall der Anschwemmfiltration, der Kuchenfiltration, bestimmt die Struktur des Filterkuchens den Prozeßverlauf, während das eigentliche Filtermittel nach einer Anfangsphase nur noch eine Stützfunktion hat. Der Ort der Feststoffabscheidung ist ein weiteres wesentliches Merkmal für die Unterscheidung von Filtrationsprozessen. Bei der Oberflächenfiltration erfolgt die Abscheidung des Feststoffes an der Oberfläche des Filtermittels durch Siebwirkung, d.h. auf Grund der Größenverhältnisse zwischen Feststoffteilchen und Poren des Filtermittels. Demgegenüber dringen bei der Tiefenfiltration die Feststoffteilchen in das Filtermittel ein und lagern sich im Innern desselben ab.

Die in der Kartusche einsetzbaren Adsorbenzien können an sich alle anorganischen oder organischen Adsorbenzien sein, die eine ausreichende Adsorptionskapazität für das im PMU enthaltene natürliche Gemische konjugierte Oestrogene aufweisen. Als Adsorbens für das erfindungsgemäße Verfahren sind daher polymere Adsorberharze, Kieselgel, RP-Kieselgel oder vorzugsweise semipolare polymere Adsorberharze für den Einsatz in der Kartusche geeignet.

Die in der Kartusche als Adsorbens einsetzbaren hydrophobisierten Kieselgele sind z.B. an sich bekannte Umkehr-Phasen-Kieselgele (= Reverse-Phase-Kieselgele, abgekürzt "RP-Kieselgele"), das sind chemisch modifizierte Kieselgele, die hydrophobe funktionelle Gruppen bzw. Liganden tragen. So eignen sich beispielsweise silanisierte RP-Kieselgele, welche als hydrophobe funktionelle Gruppen n-Octadecyldimethylsilyloxy-, n-Octyldimethylsilyloxy- oder Dimethylhydroxysilyloxy-Reste enthalten. Geeignet sind z.B. silanisierte Kieselgele mit mittleren Korngrößen von 15 bis 500 µm. Als besonders zweckmäßig hat sich Dimethylhydroxylsilyloxy-Reste enthaltenes Kieselgel mit einer mittleren Korngröße im Bereich von 0,05 - 0,3 mm, beispielsweise das "Kieselgel 60/Dimethylsilanderivat" der Firma Merck, erwiesen. Zweckmäßigerweise kann die Harnflüssigkeit durch die das Kieselgel enthaltende Kartusche mit einer solchen Durchlaufgeschwindigkeit geleitet werden, daß die Kontaktzeit zur Adsorption des Oestrogengehaltes ausreicht. Geeignet sind beispielsweise Durchlaufgeschwindigkeiten, welche einem Durchlauf von 5 bis 20 Vol.-Teilen PMU pro 1 Vol.-Teil Kieselgel/Stunde entsprechen. Die Adsorption wird bevorzugt bei Raumtemperatur durchgeführt. Zweckmäßig kann die Durchlaufgeschwindigkeit der Harnflüssigkeit durch den Reaktor durch Arbeiten bei geringem Überdruck (durch die Leistung der Pumpe geregelt) gesteuert werden. Die Menge an anzuwendendem hydrophobisiertem Kieselgel kann je nach Art des verwendeten Kieselgels und Menge des Feststoff-gehaltes in der gesammelten Harnflüssigkeit variieren. Bei Verwendung von vorfiltriertem, d.h. von Schleim- und Feststoffen befreitem, PMU kann beispielsweise ein Volumenteil hydrophobisiertes Kieselgel mit bis zu 80 Volumen-Teilen vorbehandeltem PMU beladen werden, ohne daß merkliche Mengen Oestrogen in der abfließenden Harnflüssigkeit nachweisbar sind.

Die in der Kartusche als Adsorbens einsetzbaren semipolaren polymeren Adsorberharze sind vorzugsweise poröse organische nichtionische Polymere, welche im Gegensatz zu unpolaren hydrophoben polymeren Adsorberharzen eine intermediäre Polarität (= z.B. mit einem Dipolmoment der aktiven Oberfläche des Harzes im Bereich von 1,0 bis 3,0, insbesondere I,5 bis 2,0, Debye) und eine etwas hydrophylere Struktur besitzen, beispielsweise Polycarbonsäureesterharze. Zweckmäßig werden makroporöse semipolare Harze mit vorzugsweise makroretikularer Struktur und mit durchschnittlichen Porendurchmessern im Bereich von 50 bis 150, vorzugsweise 70 bis 100, Angström und einer spezifischen Oberfläche im Bereich von 300 bis 900, vorzugsweise 400 bis 500, m²/g eingesetzt. Als besonders geeignet erweisen sich makroporöse quervernetzte aliphatische Polycarbonsäureesterharze, insbesondere quervernetzte Polyacrylesterharze wie z.B. Amberlite XAD-7^{R} der Fa. Rohm und Haas. Zweckmäßigerweise kann die Harnflüssigkeit durch die das Adsorberharz enthaltende Kartusche mit solcher Durchlaufgeschwindigkeit geleitet werden, daß die Kontaktzeit zur Adsorption des Oestrogengehaltes ausreicht. Geeignet sind beispielsweise Durchlaufgeschwindigkeiten, die einem Durchlauf von 3 bis 10, vorzugsweise 5 bis 7, Vol.-Teilen PMU pro 1 Vol.-Teil Adsorberharz/Stunde entsprechen. Die Adsorption wird bevorzugt bei Raumtemperatur durchgeführt. Zweckmäßig kann die Durchlaufgeschwindigkeit der Harnflüssigkeit durch den Reaktor durch Arbeiten bei geringem Überdruck (durch Leistung der Pumpe geregelt) gesteuert werden. Die Menge an anzuwendendem semipolarem Adsorberharz kann je nach Art des verwendeten Adsorberharzes und Menge des Feststoff-Gehaltes in der gesammelten Harnflüssigkeit variieren. Bei Verwendung von PMU kann beispielsweise ein Volumenteil Adsorberharz, z.B. quervernetztes aliphatisches Polycarbonsäureester-Adsorberharz, mit bis zu 80 Volumen-Teilen vorbehandeltem PMU beladen werden, ohne daß merkliche Mengen Oestrogen in der abfließenden Harnflüssigkeit nachweisbar sind.

Neben den bevorzugt genannten Adsorbenzien können auch andere Adsorberharze oder Kieselgeltypen eingesetzt werden. Als Adsorberharze eignen sich dabei sowohl unpolare, semipolare und auch polare Adsorberharze. Die Menge des Harns, die über den Adsorber gepumpt werden kann, ist hierbei zuvor anhand der jeweiligen Adsorberkapazität zu bestimmen. Beispiele für verwendbare Adsorberharze sind handelsübliche Typen wie polymere Amberlite Adsorbenzien mit Styroldivinylbenzolgrundgerüsten (z.B. Typen XAD-1180, XAD-2, XAD-4, XAD-16), mit Acrylestergrundgerüsten (z.B. XAD-7) oder solche mit hochpolaren Grundgerüsten enthaltend Stickstoff und Sauerstoff (z.B. XAD-12). Andere Adsorberharze sind Dowex-Harze (Copolymere von Styrol und Divinylbenzol), wie z.B. Dowex 112, Dowex Optipore, Dowex Optipore V 493; Lewatite (vernetzte Polystyrole), z.B. Lewatit OC 1064, Lewatit OC 1066 oder Lewatit OC 1163, Polyaminanionentauscherharze, z.B. Dowex-Harze. Vorteilhafte Adsorberharze sind insbesondere XAD-7, XAD-16 (Typ HP), XAD 118 und Dowex Optipore, bevorzugt als Dowex Optipore V 493, sowie Lewatite OC 1064, OC 1066 und OC 1163.

Neben dem vorstehend beschriebenen erfindungsgemäßen Verfahren betrifft die Erfindung auch eine in diesem Verfahren einsetzbare Vorrichtung, die den einfachen Bedingungen am Einsatzort in zweckmäßiger Weise Rechnung trägt und eine sichere, produktschonende Durchführung des Verfahrens gewährleistet.

Die erfindungsgemäße Vorrichtung ist charakterisiert durch ihre Eignung zur praxisgerechten, dezentralen, d.h. insbesondere aufstallungs- oder weidenahen, Anreicherung und Stabilisierung von im Harn trächtiger Stuten (PMU) enthaltener Gemische konjugierter Oestrogene (CE) auf einem festen Träger (= Adsorbens), wobei diese Vorrichtung umfaßt:
- eine aufrecht angeordnete Kartusche, die mit einem (ständig) von Flüssigkeit umgebenen, zur Adsorption einer vordefinierten Menge des in der Harnflüssigkeit trächtiger Stuten enthaltenen Gemisches konjugierter Oestrogene (CE) geeigneten Adsorbens gefüllt ist, und wobei die Kartusche entweder a) einen fußseitig angeordneten Flüssigkeitseinlaß und einen kopfseitig angeordneten Flüssigkeitsauslaß oder b) einen kopfseitig angeordneten Flüssigkeitseinlaß und einen fußseitig angeordneten Flüssigkeitsauslaß aufweist,
- sowie vor der Kartusche in der angegebenen Reihenfolge angeordnet und durch Schlauchleitungen miteinander verbunden
- eine Pumpe, einen Strömungsmesser und einen Durchflußmesser.

Die erfindungsgemäße Vorrichtung ist zur Durchführung des obenbeschriebenen Verfahrens zur praxisgerechten, dezentralen Anreicherung und Stabilisierung von im PMU enthaltenen konjugierten Oestrogenen (CE) bestimmt. Gewünschtenfalls kann die Vorrichtung auch zwei parallel geschaltete Kartuschen enthalten, die nacheinander bzw. im Wechsel betrieben werden können.

Die in der erfindungsgemäßen Vorrichtung verwendete Kartusche kann in bezug auf Höhe und Durchmesser in einem weiten Rahmen dimensioniert sein. Um die Handhabung in der Praxis allerdings nicht zu beeinträchtigen, sollte die Kartusche nach Größe und Gewicht so dimensioniert sein, daß sie von einer normalkräftigen Person, z.B. beim Auswechseln beladener Kartuschen gegen frische unbeladene Kartuschen, noch gut manuell gehandhabt und getragen werden kann. Beispielsweise sollte die Kartusche daher etwa so dimensioniert sein, daß die Kartusche Innenabmessungen für das Adsorbensbett aufweist, die es erlauben 30 bis 50 1 Adsorbens aufzunehmen, wobei die Innenhöhe insbesondere etwa im Bereich von 80 bis 120 cm liegen wird. Der Innendurchmesser der Kartusche wird im allgemeinen bei 10 bis 25 cm liegen. Der Fachmann wird hierbei in der Lage sein, eine genaue Dimensionierung der Kartusche vorzunehmen, um optimale Strömungsverhältnisse beim Betrieb sicherzustellen.

Weiterhin besteht die in der erfindungsgemäßen Vorrichtung verwendete Kartusche zweckmäßigerweise aus robusten, strapazierfähigen und Chemikalien beständigen Materialien wie sie üblicherweise für die Herstellung von Geräten für die Chemische Industrie verwendet werden, z.B. schlagfestes Laborglas, Kunststoff und/oder Metall wie z.B. Stahlblech.

In der erfindungsgemäßen Vorrichtung können an sich beliebige, einfach und robust gebaute Pumpen eingesetzt werden, wie z.B. Monopumpen, Schlauchpumpen oder Membranpumpen. Beispielsweise hat sich als Pumpe für die erfindungsgemäße Vorrichtung eine Schlauchpumpe als zweckmäßig erwiesen.

In der erfindungsgemäßen Vorrichtung können an sich beliebige, einfach und robust gebaute Strömungsmesser eingesetzt werden wie z.B. Rotameter, Drehflügelströmungsmesser oder induktive Strömungsmesser. Beispielsweise hat sich als Strömungsmesser für die erfindungsgemäße Vorrichtung ein Rotameter mit Schwimmkörper als zweckmäßig erwiesen.

In der erfindungsgemäßen Vorrichtung können zur Messung der über die Kartusche gepumpten Menge an Harnflüssigkeit an sich beliebige, einfach und robust gebaute Durchflußmesser, wie z.B. Wasseruhren oder induktive Durchflußmesser eingesetzt werden. Als zweckmäßiger Durchflußmesser für die erfindungsgemäße Vorrichtung hat sich beispielsweise eine Wasseruhr erwiesen.

In einer zweckmäßigen Variante der erfindungsgemäßen Vorrichtung sind zwischen Pumpe und Strömungsmesser zusätzlich wenigstens ein oder mehrere Vorfilter, beispielsweise Tiefenfilter oder Anschwemmfilter, zwischengeschaltet. Bei den Filtern kann es sich z.B. um einen Tiefenfilter mit Sandbett oder eine handelsübliche Filterkartusche, oder handelsübliche Anschwemmfilter oder Plattenfilter, Kerzenfilter, Filterbeutel oder auch Filtrationsrohre handeln. Dem Vorfilter kann gegebenenfalls noch ein Partikelabscheider nachgeschaltet sein, z.B. im Falle eines Sandbetts als Vorfilter ein nachgeschalteter Sandabscheider. Die Filter können in Varianten der Vorrichtung einzeln oder mehrere auch in beliebiger Kombination miteinander, z.B. in Serie hintereinander und/oder parallel geschaltet sein. Eine vorteilhafte Ausgestaltung dieser Varianten zeichnet sich dadurch aus, daß der Vorfilter ein Modul aus zwei parallel geschalteten Vorfiltern ist, die im Wechsel einzeln betrieben und auch bei laufendem Betrieb der Vorrichtung gegen einen frischen Vorfilter ausgetauscht werden können. Eine weitere vorteilhafte Ausgestaltung dieser Varianten zeichnet sich dadurch aus, daß die Filteranlage aus einem Modul von in Serie geschalteter Vorfiltrationseinheit aus Vorfilter (z.B. Filterbeutel) und einem oder zwei Tiefbettfiltern und daran anschließend zwei parallel geschalteten Absolut-Filtern (z.B. Membranfilter) besteht, wobei die Absolut-Filter im Wechsel einzeln betrieben und auch bei laufendem Betrieb der Vorrichtung gegen einen frischen Filter ausgetauscht werden können. Es ist weiterhin gegebenenfalls zweckmäßig, daß in der erfindungsgemäßen Vorrichtung zur Kontrolle der ordnungsgemäßen Funktion des Vorfilters jeweils ein Manometer zwischen Pumpe und Vorfilter sowie ein Manometer im Bereich nach dem Vorfilter, vorzugsweise erst hinter dem Strömungsmesser angeordnet, zwischengeschaltet ist.

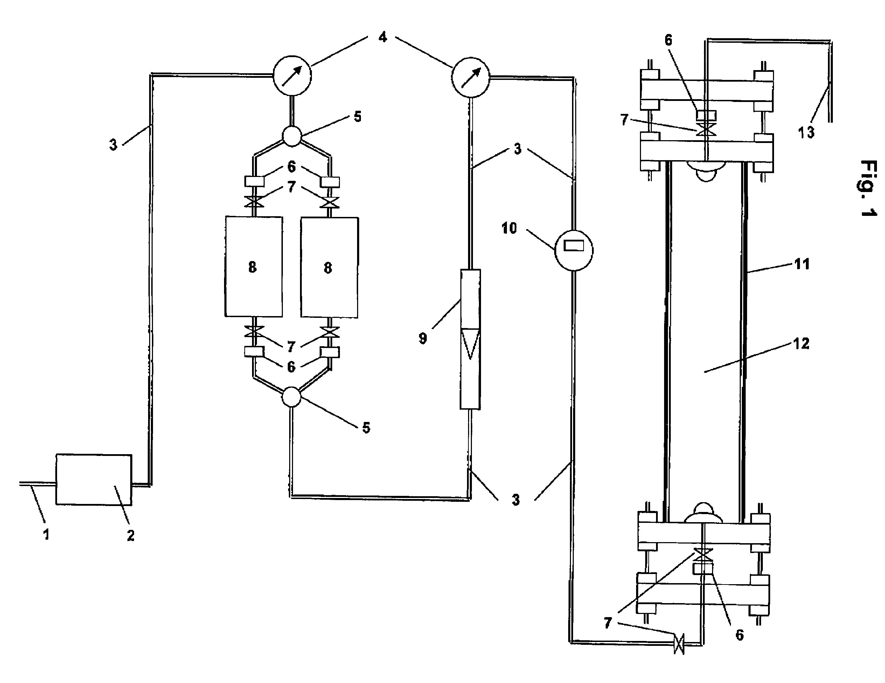

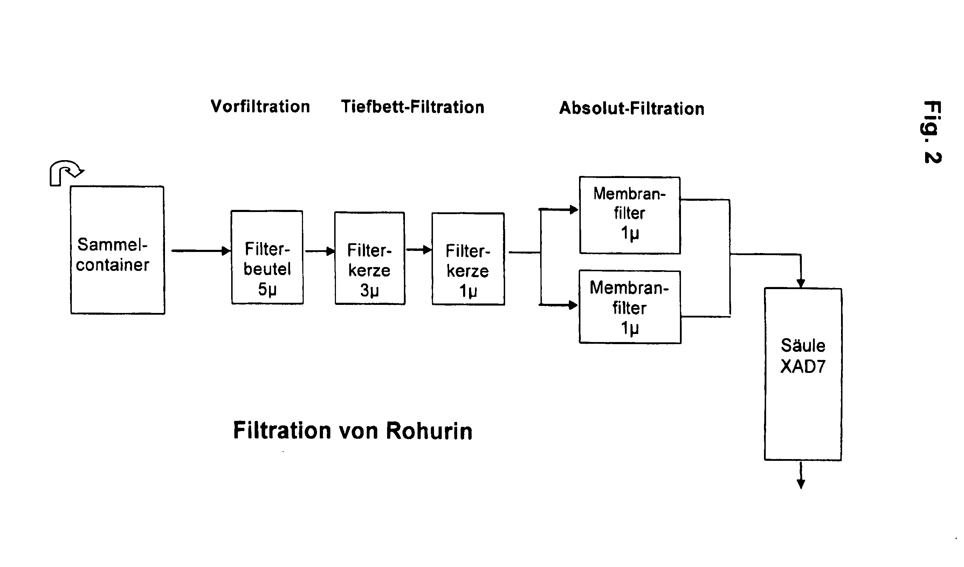

Zur weiteren Erläuterung der erfindungsgemäßen Vorrichtung in bevorzugter Ausgestaltung dient die Figur 1, die eine schematische Vorrichtung zur Stabilisierung und Anreicherung von CE aus PMU zeigt. Eine schematische Darstellung der Verfahrensweise einer Variante der Filtration von Rohurin ist in Fig. 2 wiedergegeben.

Das erfindungsgemäße Verfahren und die erfindungsgemäße Vorrichtung weisen eine Reihe von Vorteilen auf, von denen nachfolgend die wichtigsten kurz genannt seien. Die Anreicherung der CE, d.h. die Beladung der Adsorbersäulen kann direkt am Ort der Sammlung durchgeführt werden. Es entfällt dadurch der tägliche Transport von großen Mengen Harnflüssigkeit zu einer zentralen Aufarbeitungsstelle. Die Beladung kann über eine längere Zeit bis zu mehreren Wochen kontinuierlich erfolgen, bis die Adsorbersäule gesättigt ist; z.B. beim Adsorberharz XAD-7 beträgt die Menge des adsorbierten Harns ca. 35 Bettvolumen. Der von CE befreite Restharn bleibt an Ort und Stelle, wo für die Entsorgung ohnehin geeignete Einrichtungen vorhanden sind. Transportiert wird die beladene Säule, d.h., nur ca. 1/35 des ursprünglichen Gewichtes der Harn-Flüssigkeit. Der Transport kann in größeren Zeitintervallen erfolgen. Der Transport kann auch längere Zeit, z.B. durchaus bis zu einigen Wochen, in Anspruch nehmen. Wegen der guten Stabilität der CE am Adsorbersäule ist keine Zersetzung zu erwarten. Es entfällt die Behandlung von großen Mengen Harn durch Separator und Ultrafiltrations-Anlage, da die Abtrennung von im Ausgangsharn eventuell vorhandenen Schleimstoffen und Sedimenten über robuste und wartungsfreie Anschwemm- oder Tiefenfilter (z.B. Sandfilter, Filterkartusche, Anschwemmfilter, Plattenfilter, Kerzenfilter, Filterbeutel oder Filtrationsrohre) erfolgt, welche erforderlichenfalls leicht ausgetauscht werden können.

Die nachfolgenden Figuren und Beispiele sollen die Erfindung näher erläutern, ohne jedoch ihren Umfang zu beschränken.

### Figuren

### Fig . 1

Schematische, vorteilhafte Vorrichtung zur Stabilisierung und Anreicherung von CE aus PMU; die Elemente der Vorrichtung haben folgende Bedeutung: (1) = Zulauf der Harnflüssigkeit aus dem Vorratsbehälter; (2) = Pumpe, z.B. Schlauchpumpe; (3) = Verbindungsleitungen (4) = Manometer; (5) = Dreiwegehahn; (6) = Schnellkupplungen (7) = Verschlußventile; (8) = Filter; (9) = Strömungsmesser mit Schwimmkörper (Rotameter); (10) = Durchflußmesser, z.B. Wasseruhr; (11) = Kartusche, Säule; (12) = Adsorberbett; (13) = Ablauf

### Fig. 2

Schematische Darstellung einer Verfahrensweise zur Filtration von Rohurin, bei der die Filteranlage aus einem Modul von in Serie geschalteter Vorfiltrationseinheit aus Vorfilter (z.B. Filterbeutel) und einem oder zwei Tiefbettfiltern und daran anschließend zwei parallel geschalteten Absolut-Filtern (z.B. Membranfilter) besteht.

### Beispiele

### Beispiel 1:

Allgemeine Arbeitsvorschrift zur Anreicherung und Stabilisierung von im Harn trächtiger Stuten (PMU) enthaltenen konjugierten Oestrogenen (CE) direkt am Sammelort des PMU durch Adsorption an ein in einer Kartusche enthaltenes Adsorbens.

Der gesammelte PMU wird hierzu jeweils über einen mit einem Sieb bestückten Trichter in ein Vorratsgefäß gefüllt. Das Sieb weist eine grobe Maschenweite auf, und dient lediglich zur Abtrennung grober mechanischer Verunreinigungen wie Stroh, Heu etc..

Das Verfahren wird nachfolgend beispielhaft unter Verwendung einer Adsorbersäule (als Kartusche) mit einem semipolaren Adsorberharz erläutert. Werden Kartuschen mit anderen Adsorbenzien eingesetzt, richtet sich gegebenenfalls die Handhabung und insbesondere aber die Aufarbeitung der beladenen Kartusche nach der Art des jeweiligen Adsorbens. Der Beladevorgang und das abschließende Waschen der Kartusche am Urin-Sammelort wird im wesentlichen wie unter A) und B) beschrieben beibehalten oder gegebenenfalls nur geringfügig an etwaige besondere Verhältnisse vor Ort oder das jeweilige Adsorbens angepaßt.

### A) Adsorption des Oestrogengehaltes des PMU an ein Adsorbens

Die Adsorption des CE-Gehaltes des PMU wird in einer Adsorptionsanlage wie beispielhaft in Fig. 1 skizziert durchgeführt. Eine bezogen auf die Kartuschen- bzw. Adsorbersäulen-Kapazität vorgegebene Gesamtmenge PMU wird bei Umgebungstemperatur, ggf. nach vorherigem Sieben und ggf. Filtration, insgesamt oder in Teilen mit einer vorgegebenen Durchflußgeschwindigkeit über eine mit einem Adsorbens befüllte Säule bzw. Kartusche gepumpt. Als Adsorber kann z.B. ein in Wasser gequollenes semipolares Polyacrylester-Adsorberharz (= Amberlite XAD-7 der Fa. Rohm und Haas, Korngröße 0,3 bis 1,2 mm, Dipolmoment 1,8 Debye, durchschnittlicher Porendurchmesser 80 Angström, spezifische Oberfläche ca. 450 m²/g trocken) oder andere geeignete Adsorbenzien eingesetzt werden. Nach Erreichen der vorgegebenen maximalen Belademenge PMU wird die PMU-Zufuhr auf die Säule bzw. Kartusche gestoppt und gewünschtenfalls von der beladenen Säule bzw. Kartusche auf eine parallel geschaltete freie Säule bzw. Kartusche gepumpt. Der Oestrogengehalt des PMU ist vollständig auf der so beladenen Adsorber-Säule adsorbiert.

### B) Waschen der beladenen Adsorber-Säule

Die beladene Adsorber-Säule bzw. Kartusche wird nach Abschluß des Adsorptionsvorganges fakultativ mit Wasser oder einer anderen wäßrigen Waschlösung, z.B. eine basische Waschlösung wie insbesondere verdünnte Natronlauge (z.B. 0,5-2 N NaOH) gewaschen. Hierzu wird das Waschwasser oder die Waschlösung bei Umgebungstemperatur mit einer vorgegebenen Durchflußgeschwindigkeit von z.B. ca. 5,5 Bettvolumen pro Stunde ebenfalls durch die Säule bzw. Kartusche gepumpt. Die ablaufende Waschflüssigkeit wird verworfen.

### C) Lagerung und Transport

Zum Abtransport bzw. zur Zwischelagerung der beladenen Adsorber-Säule bzw. Kartusche wird diese nach Abschluß des Adsorptionsvorganges bzw. nach dem fakultativem Waschen (z.B. mit Wasser oder mit einer anderen wäßrigen, ggf. basischen Waschlösung, wie insbesondere verdünnte Natronlauge, z.B. 0,5-2 N NaOH) beidseitig verschlossen, aus der Beladevorrichtung abgeklemmt und an einem geeigneten Ort gelagert. Die Lagerung kann bei Umgebungstemperaturen, z.B. 15 bis 30 °C in einer normalen Lagerhalle oder auch in einem Kühlraum bei bis zu etwa 4 °C herab erfolgen. Wird die Flüssigkeit in der Adsorber-Säule gegen Gefrieren geschützt, kann auch bei tieferen Temperaturen, z.B. bis hinab zu etwa -20 °C tiefgekühlt werden. Die beladenen Säulen bzw. Kartuschen werden in regelmäßigen Intervallen von einigen Tagen bis zu wenigen Wochen vom Sammel- und Lagerort zu einer zentralen Aufarbeitungsstelle transportiert, wo die chemische Aufarbeitung und Isolierung der konjugierten Oetrogene in fachkundiger Weise gewährleistet ist.

### D) Desorption der konjugierten Oestrogene von der Adsorber-Säule bzw. Kartusche

Die Aufarbeitung der beladenen Säulen bzw. Kartuschen erfolgt in einer für das jeweilige Adsorbens zur Abtrennung von Begleitstoffen und Isolierung von CE üblichen Weise. Bei Verwendung eines semipolaren Adsordberharzes vom Typ Amberlite, z.B. XAD 7, kann die Aufarbeitung wie in der internationalen Patentanmeldung WO 98/08526 erfolgen. Hierzu wird z.B. die Elutionsflüssigkeit mit geeigneter Fließgeschwindigkeit durch die auf 45 °C vorgeheizte Säule bzw. Kartusche geleitet. Das ablaufende Eluat wird in Fraktionen aufgefangen. Die erste Fraktion entspricht ca. 1 Bettvolumen, die restlichen Fraktionen betragen jeweils ca. 0,75 Bettvolumen. Die einzelnen Fraktionen werden mittels HPLC auf ihren Gehalt an Oestronsulfatsalz, Kresol und HPMF untersucht. Die erste Fraktion wird gesammelt, solange das Eluat farblos bis schwach gelb gefärbt erscheint. Diese Fraktion enthält regelmäßig nur Spuren an Oestrogensulfatsalz.

Nachdem das erste Bettvolumen an Eluat abgelaufen ist, tritt ein Farbwechsel des Eluates zu einem intensiven dunkelbraunen Farbton ein. In den nachfolgenden 2 bis 4 Fraktionen sind dann regelmäßig ca. 80 bis 98 % der gesamten auf der Säule adsorbierten Menge konjugierter Oestrogene enthalten. Die restlichen Fraktionen enthalten nur noch geringe Mengen an Oestrogensulfatsalz. Dieses ist auch deutlich an der Abnahme der Farbintensität sichtbar. Gegebenenfalls können die Restfraktionen nach Abdestillieren des Lösemittelgehaltes ebenfalls noch weiter aufbereitet und weitere CE isoliert werden.

Die, die konjugierten Oestrogene enthaltenden Hauptfraktionen weisen jeweils hohe TS-Gehalte (TS = Trockensubstanz, durch HPLC bestimmt) an Oestronsulfatsalz auf und sind von Kresol und HPMF soweit befreit, daß diese Fraktionen bereits geeignete Extrakte für eine galenische Weiterverarbeitung darstellen.

### E) Regenerierung der Adsorberharz-Säulen

Die Regenerierung der beladenen Säulen bzw. Kartuschen erfolgt in einer für das jeweilige Adsorbens üblichen Weise. Bei Verwendung eines semipolaren Adsordberharzes vom Typ Amberlite, z.B. XAD 7, kann die Regenerierung wie in der internationalen Patentanmeldung WO 98/08526 erfolgen. Zur Regenerierung wird die Säule bzw. Kartusche z.B. zunächst mit einem auf pH 12 eingestellten 50 Gew.-% Ethanol enthaltenden Ethanol/Wasser-Gemisch, dann mit 10 Gew.-%iger wäßriger Natriumcitratlösung und nochmals mit Ethanol/Wasser-Gemisches und schließlich mit destilliertem Wasser gewaschen. Die gesamte Regeneration erfolgt für das beispeilhaft angegebene Adsorberharz bei einer Temperatur von 45 °C, wobei auch hier die Regenerier- und Waschlösungen fuß- oder kopfseitig über die Säule bzw. Kartusche geleitet werden können. Die Säule bzw. Kartusche kann mehrmals, beispielsweise bis zu 40-mal, beladen und wieder regeneriert werden.

### Beispiel 2:

### Stabilitätstests mit beladenen Adsorbersäulen zur Bestimmung des desorbierbaren CE-Gehaltes

Zur Untersuchung der Säulenstabilität, d.h. der Stabilität von adsobierten CE (conjugated estrogens, konjugierte Östrogene), wurden Stabilitätstests mit den mit Ausgangsharn beladenen Adsorbersäulen durchgeführt. In üblicher Weise wurden dazu Adsorbersäulen beladen, anschließend das Adsorberharz der Säule entnommen und bei zwei Temperaturen über unterschiedliche Zeiten gelagert. Nach Ablauf der Lagerzeit wurde das Adsorbat eluiert und auf seinen CE-Gehalt und Begleitstoffe untersucht. Das erhaltene Ergebnis wurde mit dem Elutionsergebnis von Kontrollproben, d.h. Proben ohne Lagerzeit, verglichen.

### A) Versuchsdurchführung

Die Absorption wurde jeweils in einer mit semipolaren Adsorberharz des Typs XAD 7 (semipolares Polyacrylester-Adsorberharz, d.h. Amberlite XAD-7 der Fa. Rohm und Haas, Korngröße 0,3 bis 1,2 mm, Dipolmoment 1,8 Debye, durchschnittlicher Porendurchmesser 80 Angström, spezifische Oberfläche ca. 450 m²/g trocken) in bekannter Weise befüllten 31-Säule durchgeführt. Hierzu wurde eine Menge von 90 1 (= 30 Bettvolumen) zuvor ultrafiltriertem Stutenharn (Herkunft Polen) bei Raumtemperatur mit einer Durchflußgeschwindigkeit von 5 Bettvolumen pro Stunde auf die Säule aufgegeben. Der Oestrogengehalt des PMU wurde vollständig auf der so beladenen semipolaren Adsorberharz-Säule adsorbiert. Nach abgeschlossener Beladung der Säule mit dem Stutenharn wurde mit 3 1 Wasser nachgewaschen und das beladene Harz aus der Säule ausgetragen und auf 10 Proben von 300 ml aufgeteilt und diese in kleinere Säulen überführt. Eine Probe wurde verworfen.

Anschließend wurde wie folgt weiter verfahren:
1. Sofortige Eluation der Kontrollprobe, d.h. ohne Lagerung.
2. Lagerung der jeweils übrigen Proben bei 4°C oder bei 25°C.
3. Eluation der Proben 1 (4°C) und 2 (25°C) nach einwöchiger Lagerung.
4. Eluation der Proben 3 (4°C) und 4 (25°C) nach zweiwöchiger Lagerung
5. Eluation der Proben 5 (4°C) und 6 (25°C) nach fünfwöchiger Lagerung
6. Eluation der Proben 7 (4°C) und 8 (25°C) nach achtwöchiger Lagerung

Die Eluation erfolgte in an sich bekannter Weise, indem das beladene Adsorberharz zunächst mit einer wäßrigen Natriumhydroxidlösung gewaschen wurde. Nach jedem Waschschritt wurde die jeweilige Waschflüssigkeit mittels HPLC auf ihren Gehalt an Estron (als Sulfatsalz), Equilin, Kresol und HPMF untersucht.

Als Elutionsflüssigkeit wurde durch Zusatz von Natriumhydroxid alkalisch gestelltes Wasser/Ethanol-Gemisch (30 Gew.-%ig, pH-Wert 12) verwendet und die Eluation des Harzes bei einer Elutionstemperatur von 45 °C durchgeführt. Das Eluat wurde in Fraktionen gesammelt und die einzelnen Fraktionen wiederum mittels HPLC auf ihren Gehalt an Estron, Kresol und HPMF untersucht. Die erste Fraktion wurde gesammelt, solange das Eluat farblos bis schwach gelb gefärbt erschien. Diese Fraktion enthält nur Spuren an Estron (Oestrogensulfatsalz). Nachdem das erste Bettvolumen an Eluat abgelaufen ist, tritt ein Farbwechsel des Eluates zu einem intensiven dunkelbraunen Farbton ein. In den nachfolgenden 2 bis 4 Fraktionen sind dann ca. 80 bis 98 % der gesamten auf der Säule adsorbierten Menge konjugierter Oestrogene enthalten. Die restlichen Fraktionen zur Regenerierung des Harzes (bei jeweils 45 °C in der angegebenen Reihenfolge mit 50 Gew.-%igem Ethanol mit pH 12, 10 Gew.-%iges Natriumcitrat und Wasser) enthalten nur noch geringe Menge an Oestrogensulfatsalz. Dieses ist auch deutlich an der Abnahme der Farbintensität sichtbar.

### B) Ergebnisse

Die für die Säulenstabilitätstests erhaltenen Ergebnisse für die einzelnen Proben sind in den Tabellen I.0 bis I.9 wiedergegeben bzw. in Tabelle I.10 zusammengefaßt, und sollen nachfolgend noch weiter erläutert werden.

Die Beladung der Säule bzw. Kartusche mit Harn, siehe Tabelle I.0, sowie die Eluation der Kontrollsäule, Tabelle I.1, zeigten einen normalen Verlauf der Adsorption und Desorption. Es wurden zwar erhöhte Werte für Kresol und HPMF im Eluat gefunden, was jedoch weitgehend auf eine nicht vollständig laminare Strömung in der Säule bzw. auf den mit fast 1000 mg/l sehr hohen Kresolwerten des Ausgangsharns zurückzuführen sein dürfte.

Die Tabellen I.2 bis I.9 geben die Ergebnisse für die Eluation nach ein-, zwei-, fünf- bzw. achtwöchiger Lagerung wieder. Grundsätzlich ist festzustellen, daß die adsorbierten Hormone (CE) in der Regel quantitativ desorbiert werden. Gelegentlich, z.B. insbesondere nach nur einwöchiger Lagerzeit bei 25°C, wird eine quantitative Desorption erst bei der 50% ethanolischen Regeneration festgestellt, wobei es sich hierbei jedoch nur um die Desoprtion von Restmengen der CE handelt und der größte Teil bereits zuvor in der eigentliche Desorption isoliert werden konnte. Eine Abhängigkeit der Desorption von Lagerzeit und Temperatur scheint nicht oder allenfalls nur bedingt gegeben zu sein, bspw. sofern eventuell nicht optimale Strömungsverhältnisse in den kleinen Säulen eine größere Rolle spielen können. Das Eluat ist aber in allen Fällen weitgehend Kresol- und HPMF-frei. Zur Erzielung optimaler Ergebnisse sollte somit unabhängig von der Lagerzeit auf eine optimale Säulendurchströmung geachtet werden. Die Säulen bzw. Kartuschen sollten daher ständig unter Flüssigkeit stehen, d.h. ein Trockenlaufen ist zu vermeiden.

Die Ergebnisse nach fünfwöchiger Lagerung, Tabellen I.6 bis I.7, zeigen für eine Temperatur von 4 °C einen guten Eluationsverlauf; für T = 25 °C treten allerdings noch Hormonanteile im Regenerat auf. Die HPMF- und Kresolgehalte im Eluat sind in beiden Fällen sehr gering.

Überraschenderweise werden auch nach sehr langer Lagerung sehr gute Ergebnisse erzielt, wie dieses für die achtwöchige Lagerung mit exzellenten Ergebnissen in den Tabellen I.8 bis I.9 gezeigt ist. Die Elutionspeaks für Estron und Equilin sind sehr steil bzw. scharf, d.h. das Volumen des CE-Eluates ist klein. Die Kresol- und HPMF-Werte gehen gegen Null.

Tabelle I.10 gibt alle Versuche in einem übersichtlichen Zusammenhang wieder. Die Summierung z.B. des Estrongehaltes des Eluates und Regenerates liegt in allen Fällen innerhalb von ca. 1055 mg ± 4% und entspricht damit der erwarteten Bilanzierung.

Zusammenfassend läßt sich daher festhalten, daß die einbis achtwöchige Lagerung des beladenen XAD-7 Harzes bei Temperaturen von 4 °C und 25 °C zwar Unterschiede im Eluationsverhalten, Höhe und Breite der Hormonpeaks zeigen, nicht jedoch in der Summe der insgesamt eluierten Hormone, die allesamt in Bezug auf den Estrongehalt im Bereich 1055 mg ± 4% liegen. Daraus kann gefolgert werden, daß die beobachteten qualitativen Unterschiede weitestgehend nur auf inhomogenen Strömungsverhältnissen in den verwendeten relativ kleinen Säulen beruhen und bei Verwendung von praxisgerecht dimensionierten Säulen bzw. Kartuschen leicht vermieden werden können.

### Tabelle I.0:

### Säulenversuch zur Stabilität von Säulenmaterial

### Beladung der Säule mit nativem Harn aus Polen, ultrafiltriert Adsorber XAD 7, Volumen = 3 1

Nach Aufgabe auf die Säule wird Harz ausgetragen und mit 3 1 Wasser nachgewaschen. Das Harz wird in gleiche Teile à 300ml geteilt und einem Säulenstabilitätstest unterworfen. Ein Harzpaket wird sofort eluiert, die anderen bei Raumtemperatur und im Kühlschrank gelagert.

| a) Proben | Volumen l | Estron mg/l | HPMF mg/l | Kresol mg/l | Equilin mg/l |
|---|---|---|---|---|---|
| Ausgangslösung (= nach Ultrafiltration) | | | | | |
| Kontroll-Lösung 1 | 30 | 85,4 | 67,2 | 908,0 | 50,1 |
| Kontroll-Lösung 2 | 30 | 117,7 | 96,9 | 990,4 | 70,0 |
| Kontroll-Lösung 3 | 30 | 137,6 | 105 | 997,5 | 80,2 |
| | 90 | 113,567 | 89,7 | 965,3 | 66,8 |
| | = 30 BV | = Durchschnitt | | | |

| b) Proben | Vol. I | Zeit h | Fluß l/h | Fluß BV/h | Estron mg/l | Equilin mg/l | HPMF mg/l | HPMF mg | Kresol mg/l | Kresol mg |
|---|---|---|---|---|---|---|---|---|---|---|
| Ablauf 1 | 10 | 0,7 | 15 | 5,0 | 0,0 | 0,0 | 142,6 | 1426,0 | 2,7 | 27,0 |
| Ablauf 2 | 10 | 0,7 | 15 | 5,0 | 0,0 | 0,0 | 0,0 | 0,0 | 2,2 | 22,0 |
| Ablauf 3 | 10 | 0,7 | 15 | 5,0 | 0,0 | 0,0 | 0,0 | 0,0 | 0,8 | 8,0 |
| Ablauf 4 | 10 | 0,7 | 15 | 5,0 | 0,0 | 0,0 | 148,5 | 1485,0 | 1,4 | 14,0 |
| Ablauf 5 | 10 | 0,7 | 15 | 5,0 | 0,0 | 0,0 | 150,6 | 1506,0 | 5,2 | 52,0 |
| Ablauf 6 | 10 | 0,7 | 15 | 5,0 | 0,0 | 0,0 | 153,7 | 1537,0 | 27,3 | 273,0 |
| Ablauf 7 | 10 | 0,7 | 15 | 5,0 | 0,0 | 0,0 | 153,8 | 1538,0 | 81,9 | 819,0 |
| Ablauf 8 | 10 | 0,7 | 15 | 5,0 | 0,0 | 0,0 | 155,7 | 1557,0 | 322,4 | 3224,0 |
| Ablauf 9 | 10 | 0,7 | 15 | 5,0 | 0,0 | 0,0 | 154,2 | 1542,0 | 770,5 | 7705,0 |

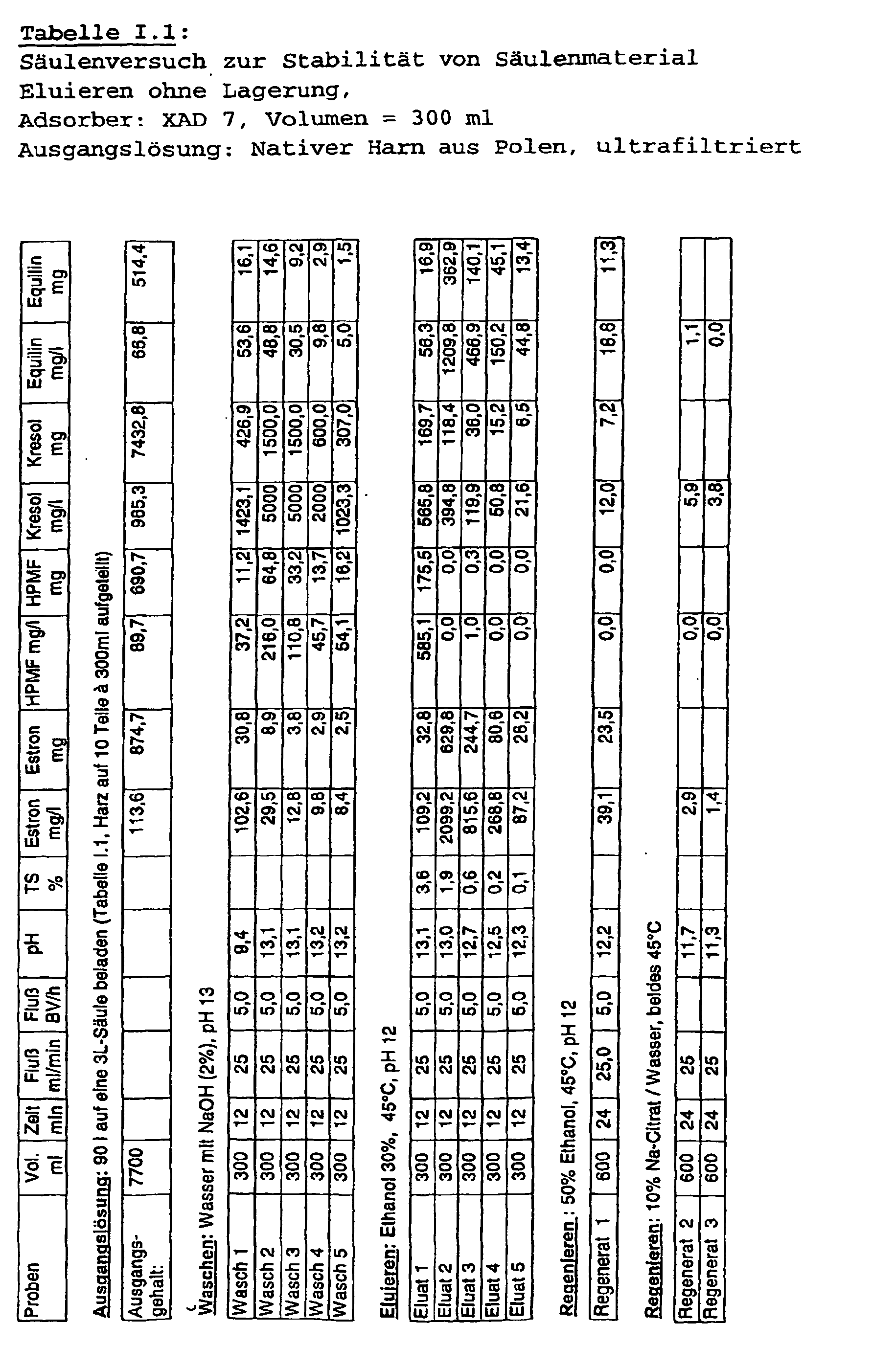

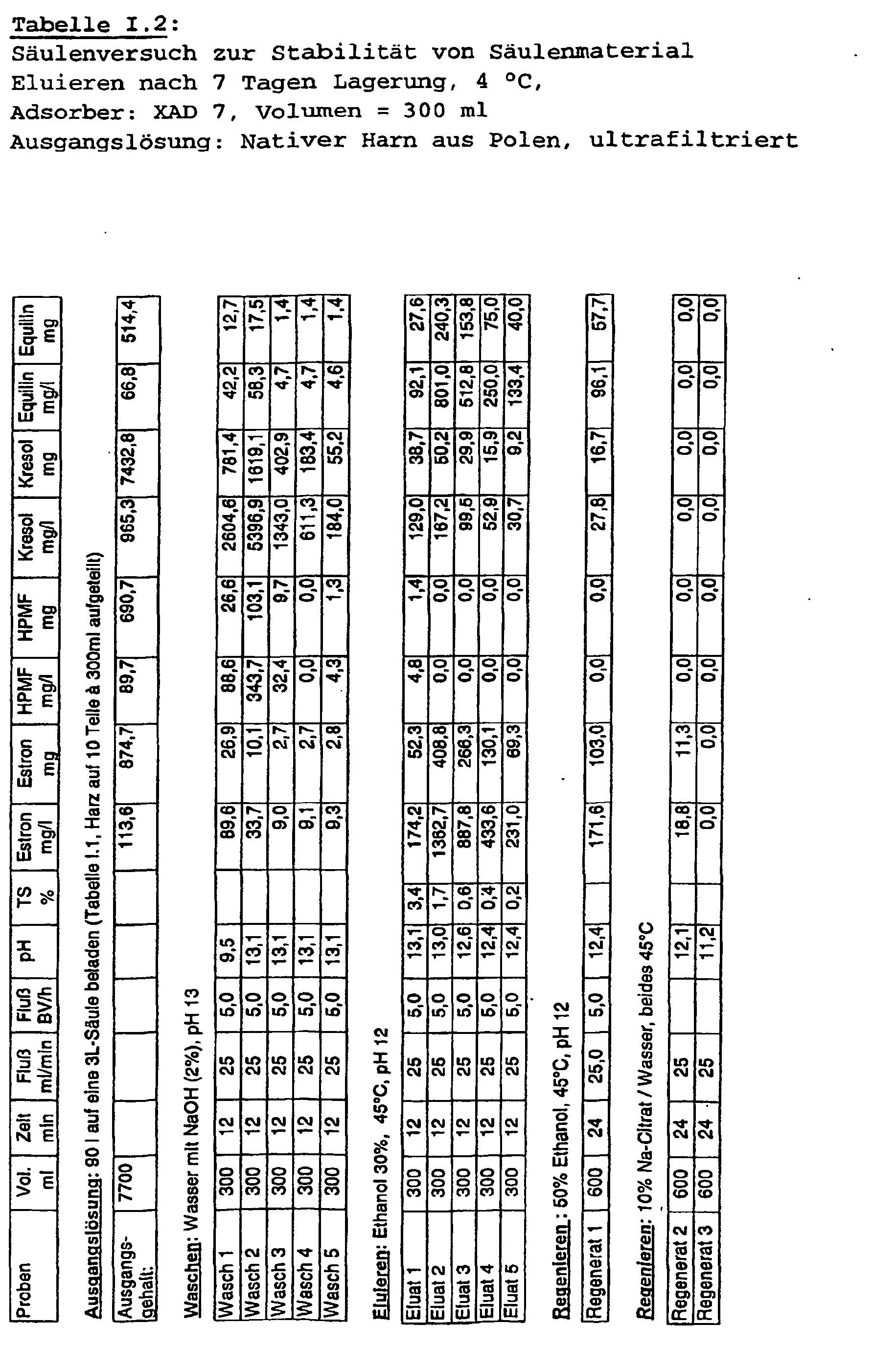

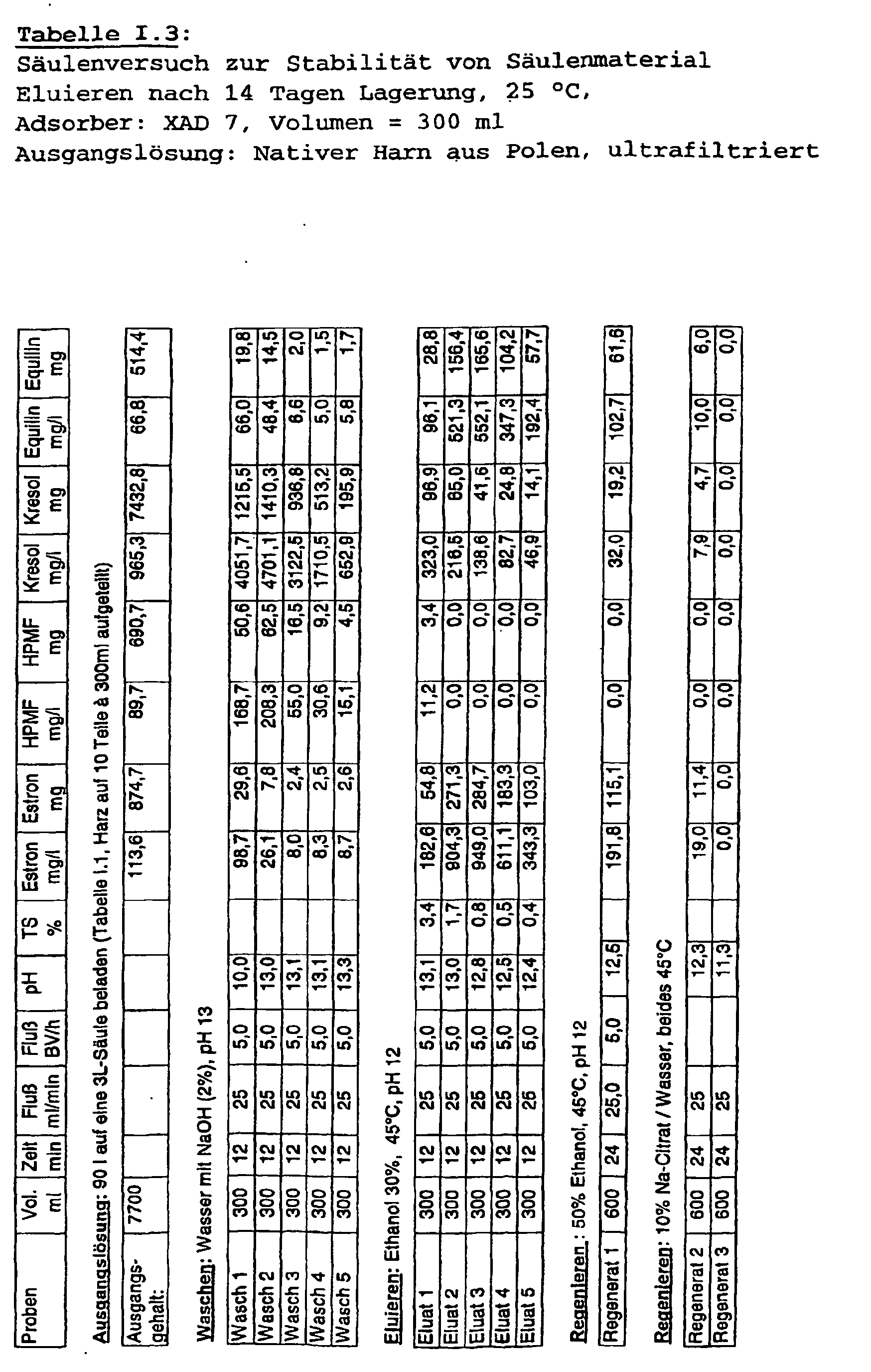

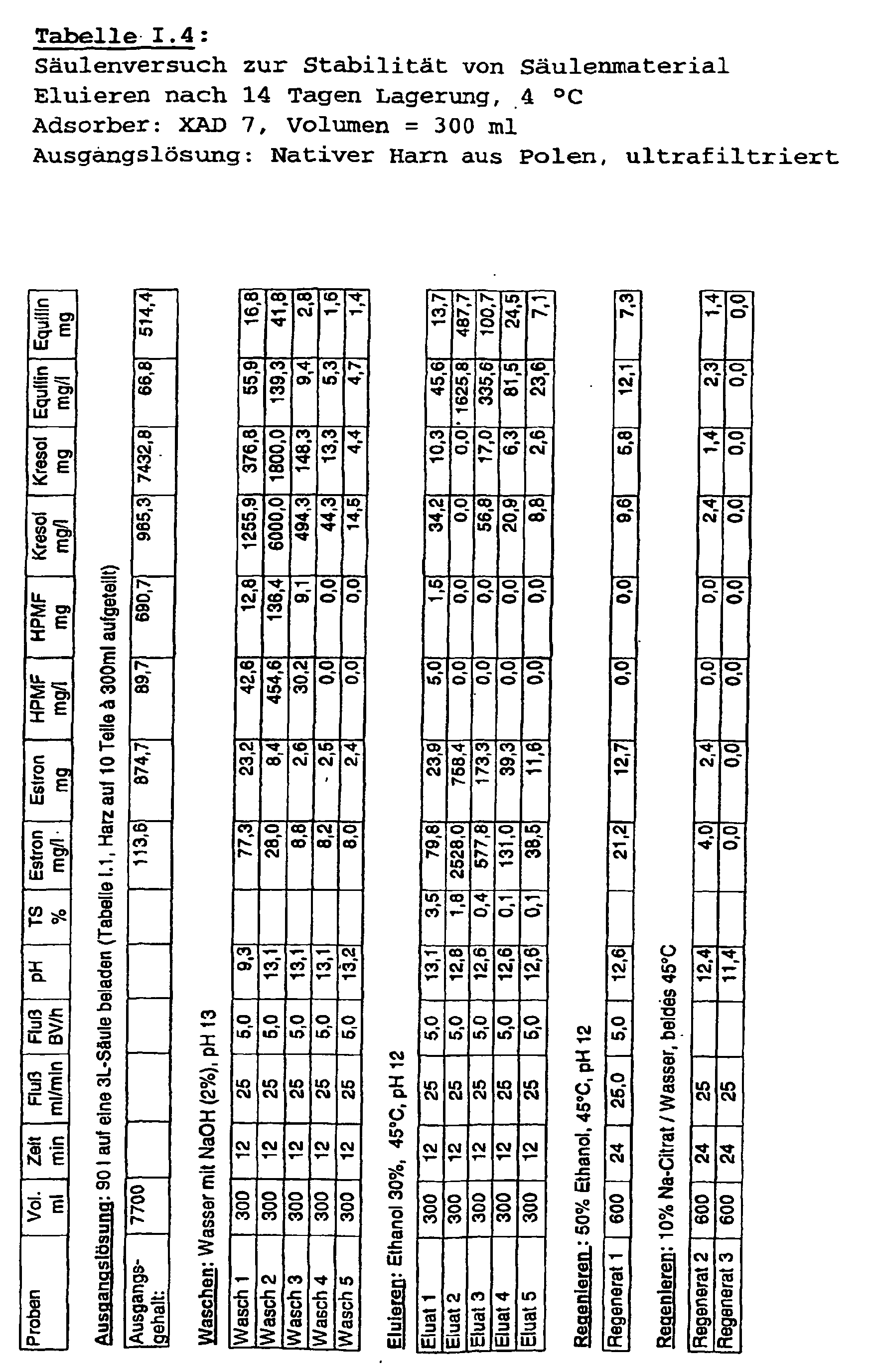

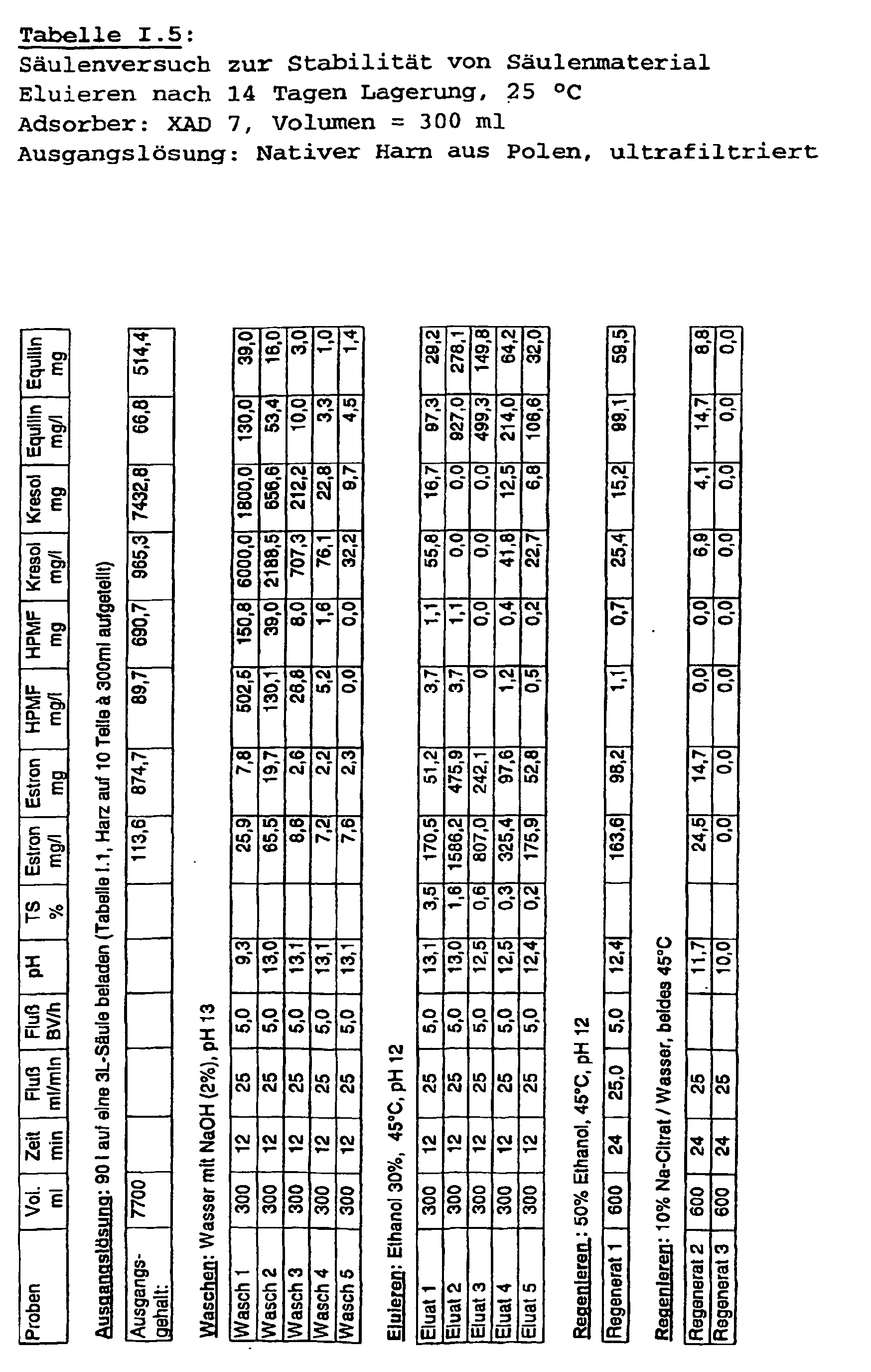

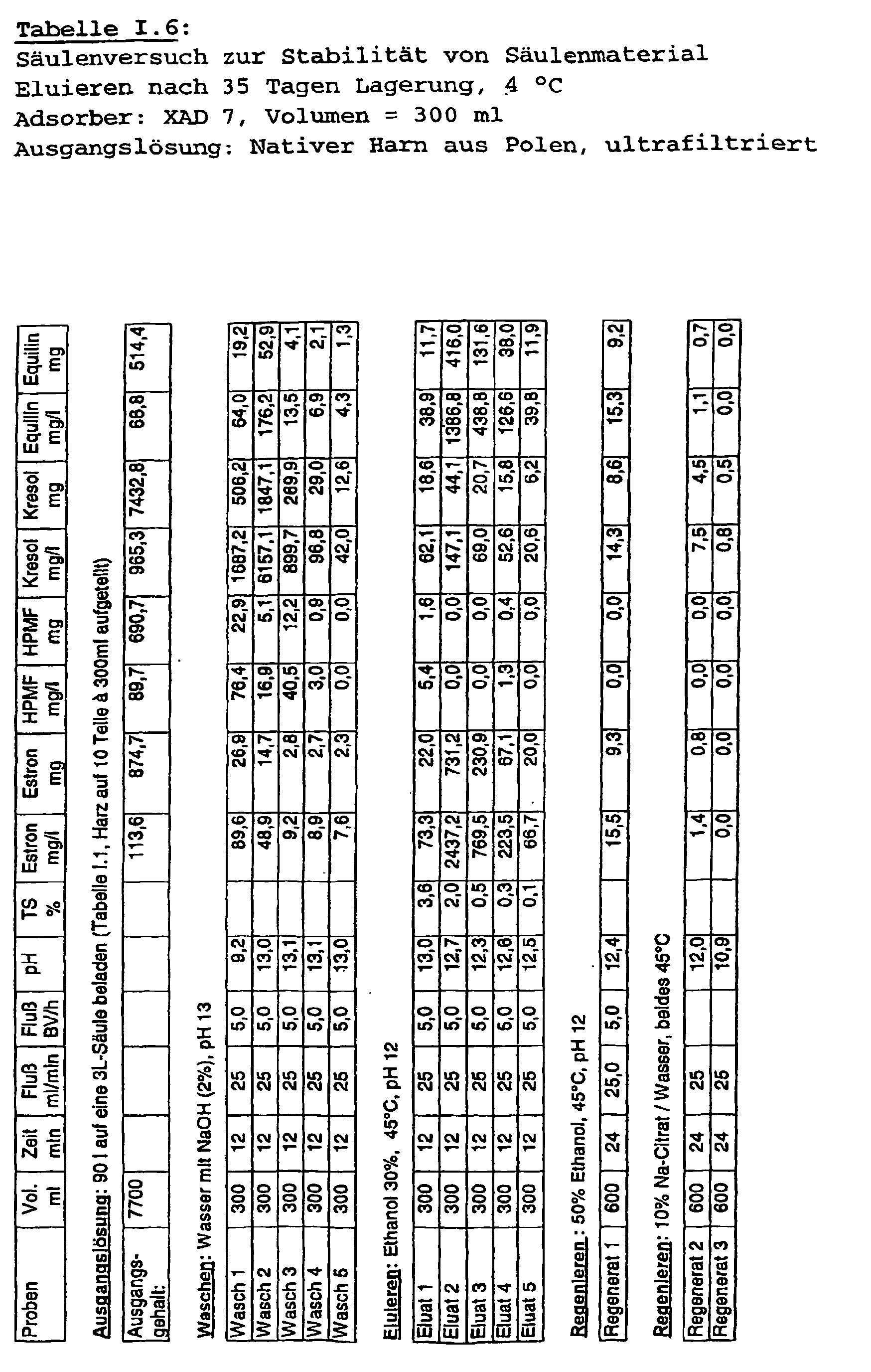

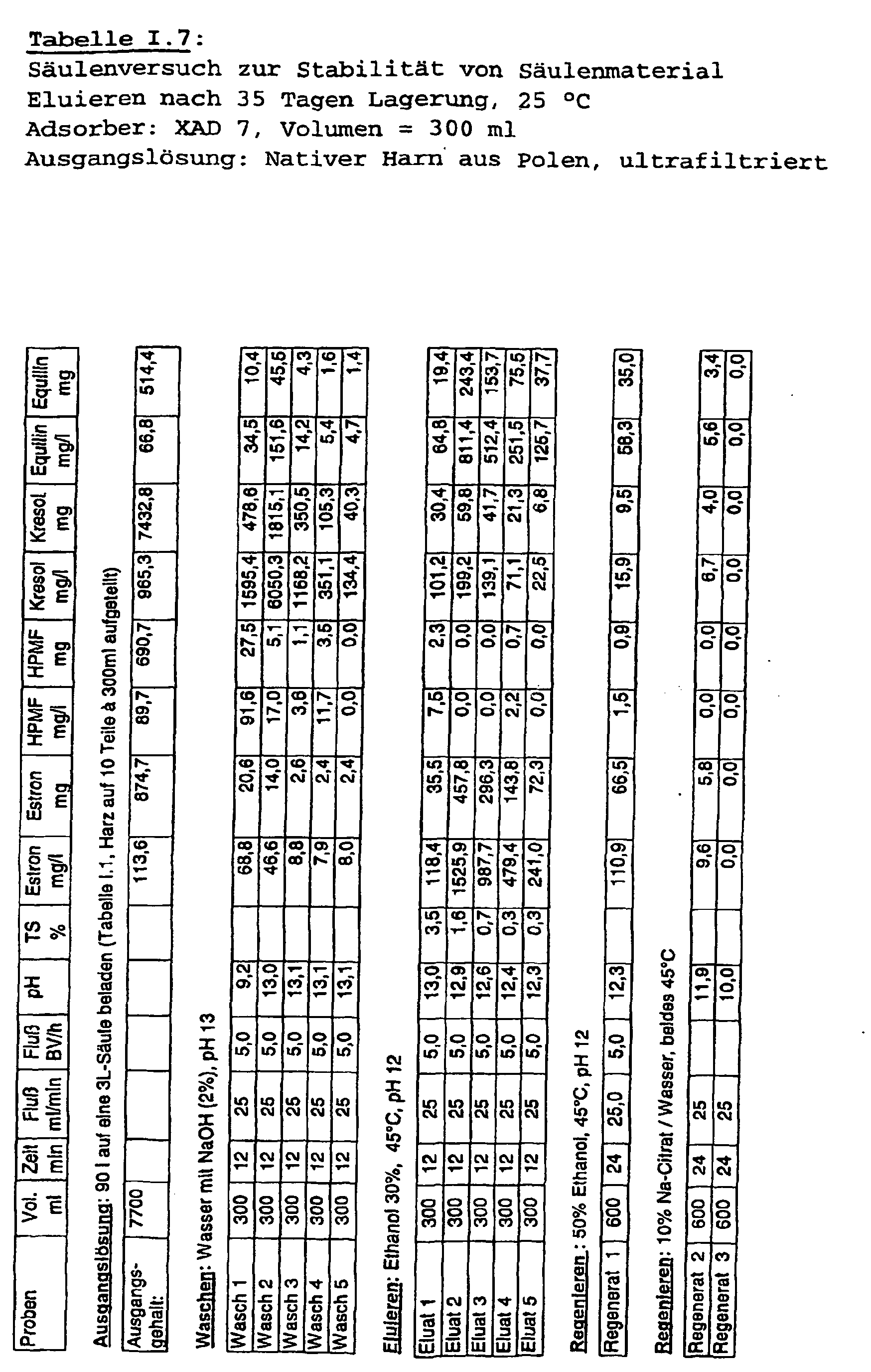

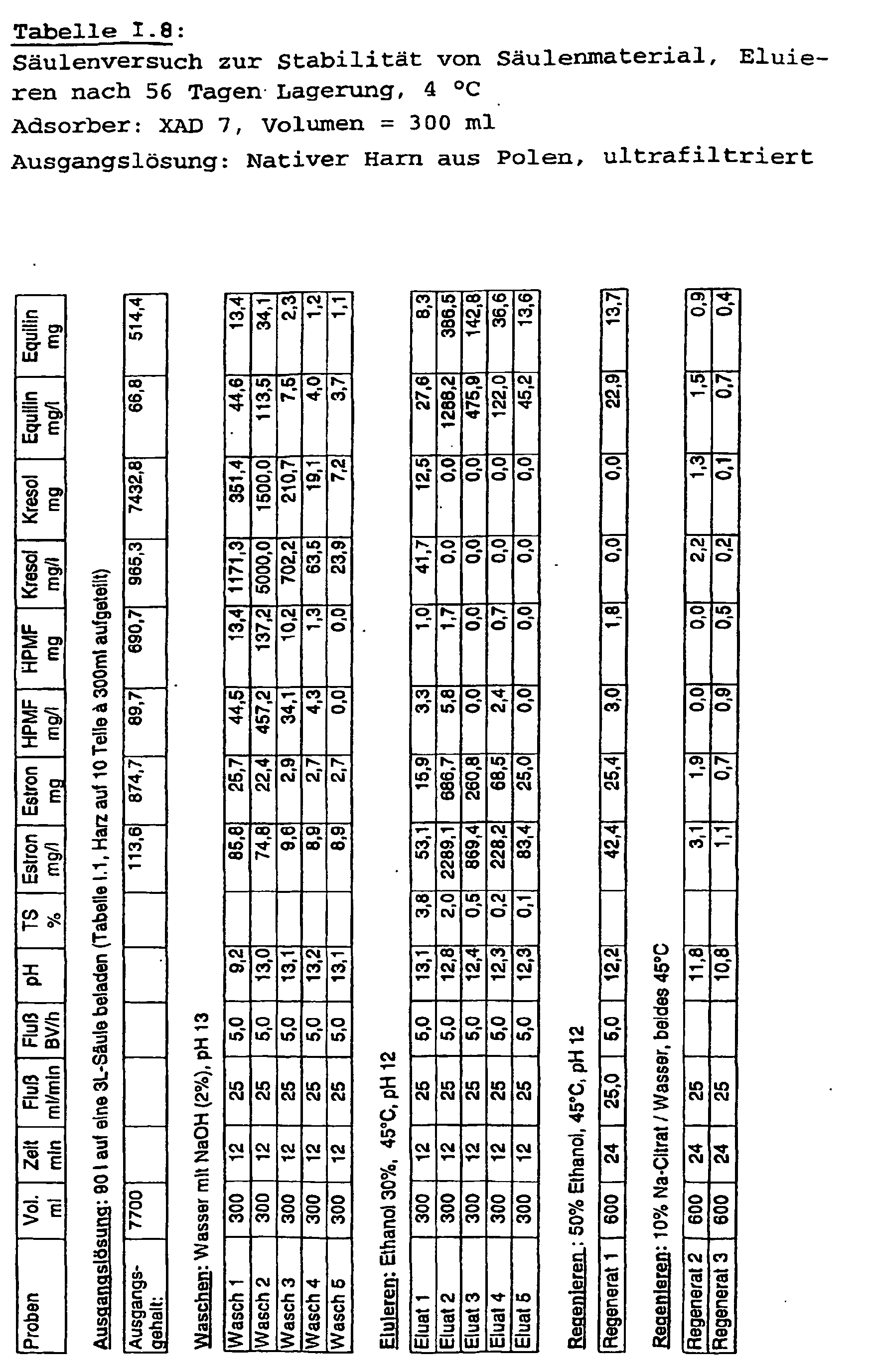

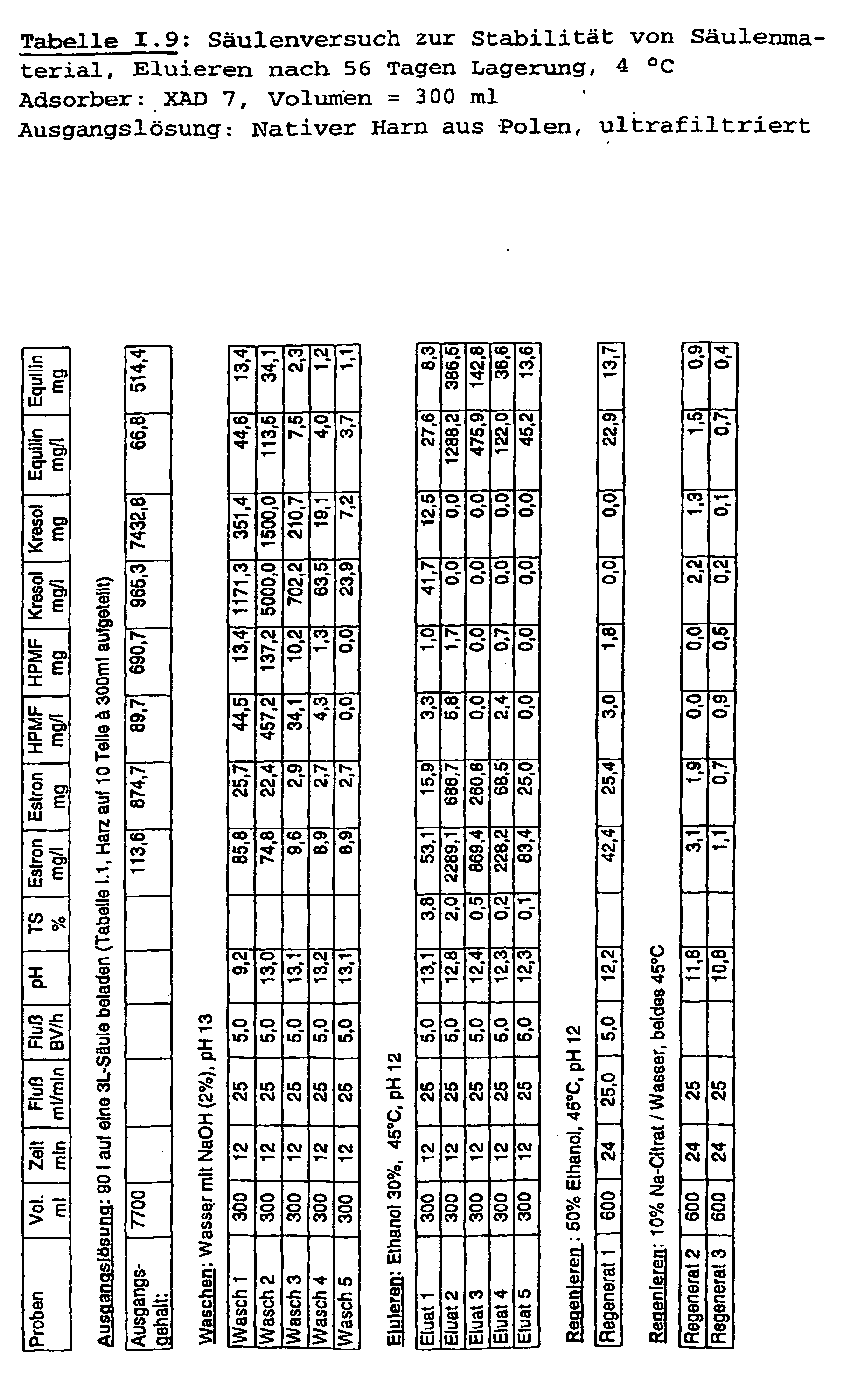

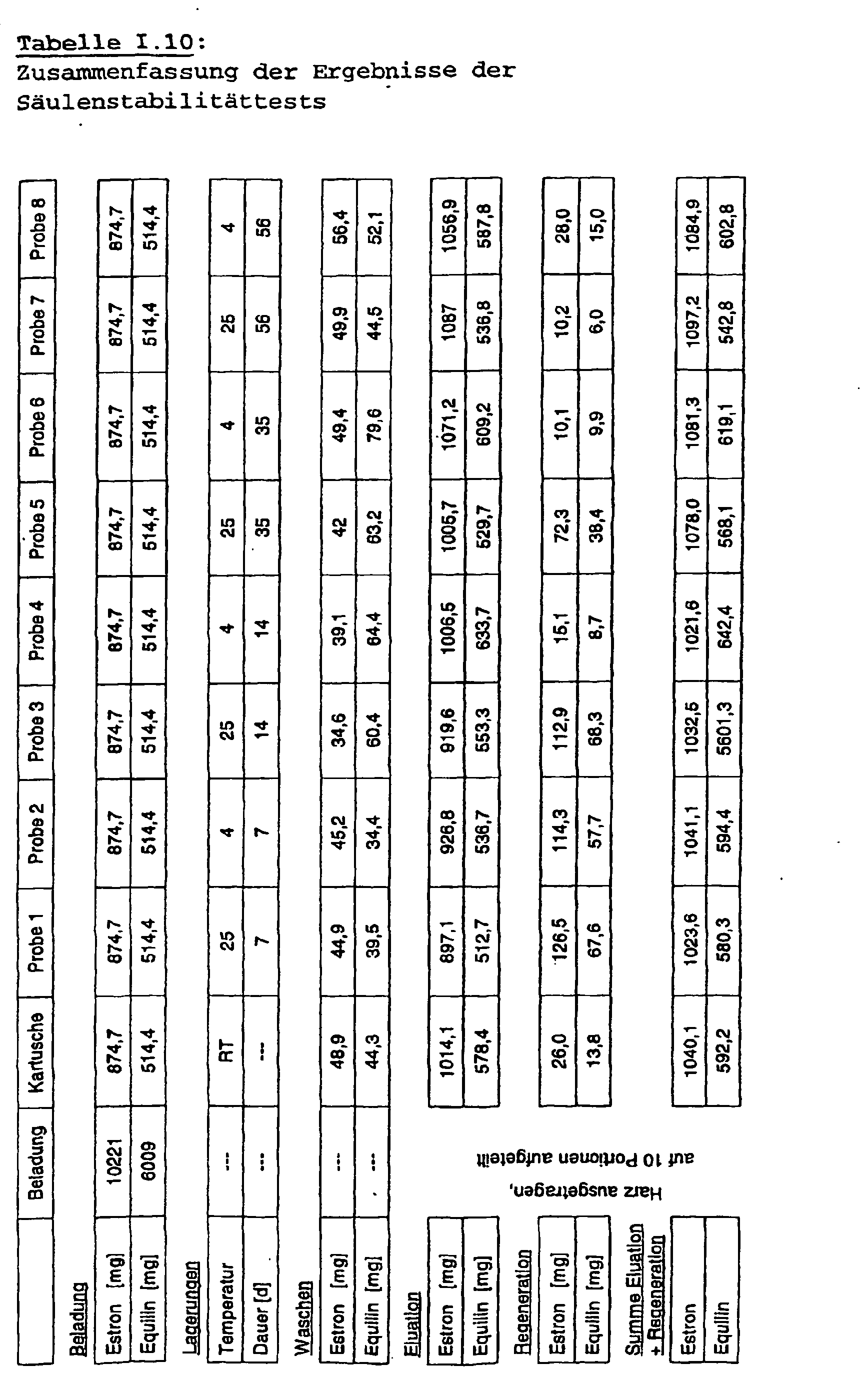

### Beispiel 3

### Stabilitätstests mit beladenen Adsorbersäulen zur Bestimmung von Keimzahlen

Die mikrobiologische Qualität und Unbedenklichkeit von natürlichen Ausgangsmaterialien zur Herstellung pharmazeutischer Präparate gewinnt in jüngerer Zeit zunehmend an Bedeutung. Es wurden daher Analysen zur Bestimmung der Keimzahlen in Proben nach einer fünf- bzw. achtwöchigen Säulenlagerung nach üblicher Methodik (Pharmacoepia .... ) durchgeführt.

Die Ergebnisse der Keimzahlbestimmungen sind in Tabelle II wiedergegeben. Die Gesamtkeimzahlen für eine Lagertemperatur von 4 °C sind als gering zu bezeichnen. Für Lagertemperatur von 25 °C weist das Säulenwaschwasser 1,2 bis 4,6 • 10⁶ Keime auf, im Eluat wurden 80 bis 170 Keime gefunden. Es sei allerdings darauf hingewiesen, daß die gelagerten SäulenAdsorbenzien zuvor weder gewaschen noch konserviert wurden, also von vorne herein einer erhöhten Keimbildungsgefahr ausgesetzt waren. Bei Lagerung ohne Kühlung empfiehlt sich daher gründliches Waschen der beladenen Säulen zumindest mit Wasser, vorteilhafter Weise aber zusätzlich mit zusätzlich stabilisierenden bzw. konservierenden Waschlösungen, z.B. Kochsalzlösung.

**Tabelle II: Stabilitätstests zur Keimzahlbestimmung**

| | Lagerzeit 5 Wochen | | Lagerzeit 8 Wochen | |
|---|---|---|---|---|
| Lagerzeit bei °C | 4 °C | 25 °C | 4 °C | 25 °C |
| Säulenwaschwasser | 200 | 4,6* 10⁶ | 150 | 1,2* 10⁶ |
| Basisches Waschwasser | < 1 | 18 | 3 | 14 |
| Ethanolisches Eluat | 20 | 80 | 3,5 | 170 |

| | | | | |
|---|---|---|---|---|
| * Angaben in KBE/ml (Kolonie bildende Einheiten) | | | | |

### Beispiel 4:

### Stabilitätstests mit tiefgekühlten, beladenen Adsorbersäulen

Jahreszeitlich und/oder regional bedingt können bei der Lagerung oder beim Transport beladener Säulen bzw. Kartuschen Temperaturen unterhalb des Gefrierpunktes auftreten, mit der unerwünschten Folge, daß der wässrige Inhalt der Säulen bzw. Kartuschen erstarrt. Entsprechende Versuche lassen darauf schließen, daß sich der Einfriervorgang negativ auf das Eluieren der wiederaufgetauten Säule bzw. Kartusche auszuwirken scheint. Sofern eine Temperierung der Säule nicht vorgenommen werden kann, oder sofern eine Lagerung bei tiefen Temperaturen stattfindet, ist es zur Verhinderung des Gefrierens empfehlenswert, die letzte Wäsche anstelle mit Wasser mit geeigneten Kältemischungen vorzunehmen.

### A) Versuchsdurchführung

Aus der Vielzahl möglicher Kältemischungen, von denen zur Erläuterung einige in Tabelle III.1 beispielhaft angegeben sind, wurde für weitere Versuche exemplarisch eine Kochsalzlösung ausgewählt. Bei einem Zusatz von 23 g NaCl pro 100 g Wasser liegt der Gefrierpunkt bei -21 °C.

Die Säulenbeladung verlief in üblicher Weise. Nach Abschluß der Beladung mit Harn erfolgte eine Spülung mit 3 Bettvolumen Wasser danach mit 2 Bettvolumen einer 33 gew.-%igen NaCl-Lösung. Anschließend wurde die Säule für 9 Tage bei -19 °C in einer Tiefkühltruhe gelagert.

**Tabelle III.1: Beispiele für flüssige wäßrige Salz- bzw. Kältemischungen**

| Erreichbare Temp. in °C | Stoff A | [g A/100 g Kältemittel] | Stoff B |
|---|---|---|---|
| -3,4 | NH₄Cl | 23 | Wasser |
| -5,3 | NaNO₃ | 43 | Wasser |
| -7,8 | BaCl₂ | 22 | Eis |
| -10 | NaCl | 26 | Wasser |
| -12 | CaCl₂ • 6 H₂O | 71 | Wasser |
| -16 | NH₄SCN | 57 | Wasser |
| -19 | (NH₄)₂SO₄ | 38 | Eis |
| -21 | NaCl | 23 | Eis |
| -22 | CaCl₂ • 6 H₂O | 45 | Eis |
| -28 | NaBr | 39 | Eis |
| -33 | MgCl₂ | 22 | Eis |
| -37 | 66, 1%ige H₂SO₄ | 48 | Schnee |
| -40 | CaCl₂ • 6 H₂O | 55 | Eis |
| -55 | CaCl₂ • 6 H₂O | 59 | Eis |

### B) Ergebnisse

Auch nach 9 Tagen Lagerung bei -19 °C blieb der Säuleninhalt flüssig, und zwar ohne Anzeichen etwaiger partieller Erstarrung. Die Aufarbeitung der Säule, d.h. die basische Wäsche, Eluierung und Regenerierung verlief normal. Der Gesamthormongehalt pro TS betrug ca. 22 Gew.-%. Die Ergebnisse sind in Tabelle III.2 dargestellt.

Negative Auswirkungen des hohen NaCl-Gehaltes auf den CE-Gehalt und die Abtrennung von Kresol und HPMF waren nicht erkennbar. Zusammenfassend läßt sich daher festhalten, daß sich eine Stabilisierung von mit Harn beladenen Säulen bzw. Kartuschen mit einer flüssigen Kältemischung, z.B. mit einer wäßrigen Kochsalz-Lösung, und eine Lagerung unter Kühlung, z.B. bis herab zu etwa -20 °C, selbst über Zeiträume von 1,5 bis 2 Wochen vorteilhaft auswirkt. Der Säulen- bzw. Kartuschen-Inhalt bleibt flüssig, die Aufarbeitung der Säulen bzw. Kartuschen zur Isolierung von CE durch Waschen, Eluierung und Regenerierung verläuft normal. Negative Befunde waren auch bei hohen NaCl-Gehalten nicht feststellbar.

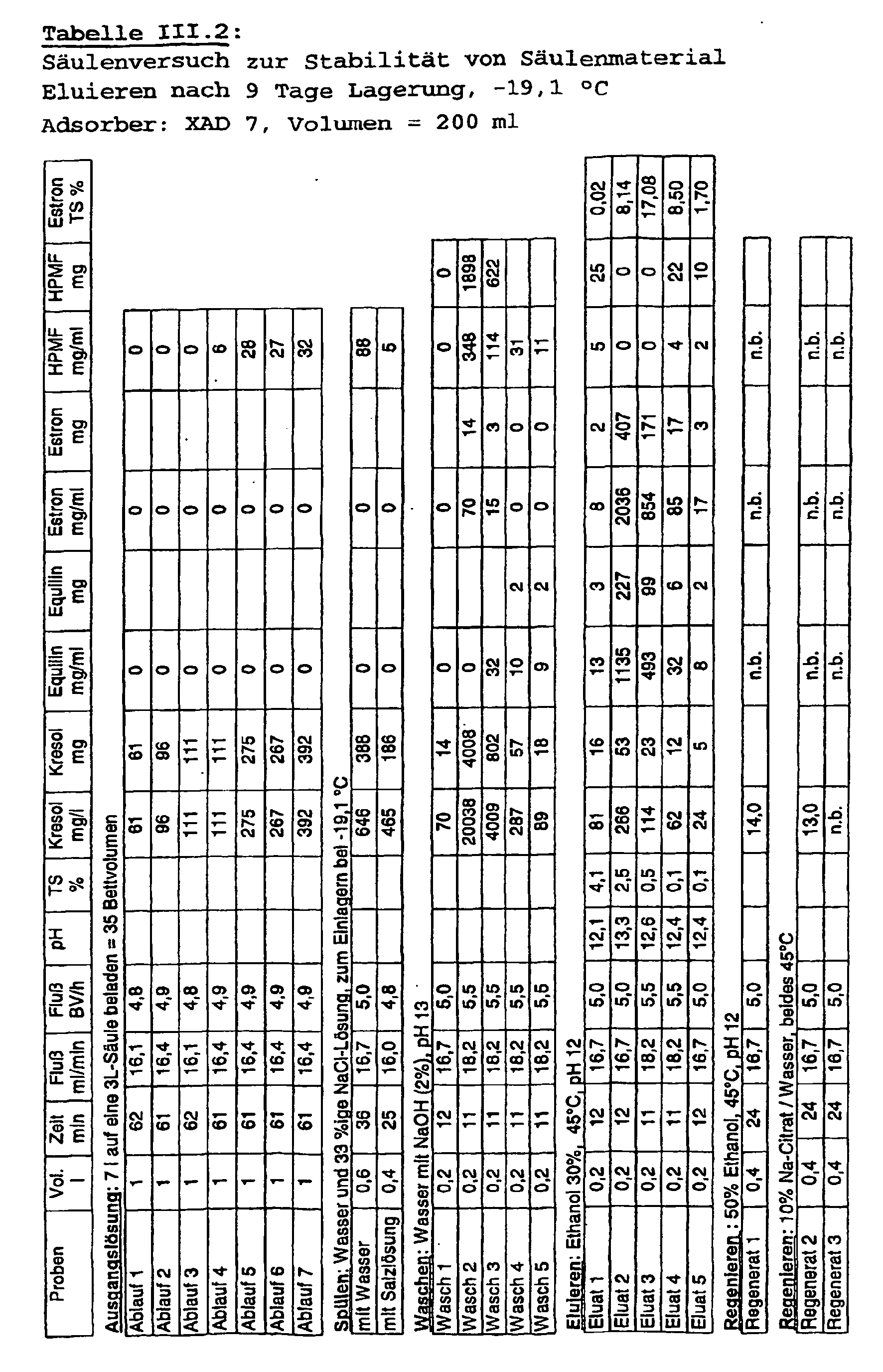

### Beispiel 5:

### Anreicherung und Stabilisierung von Harn im Praxistest

### A) Allgemeines

In einem Praxistest sollte gezeigt werden, daß bei ausreichendem Hormongehalt im Harn nicht nur im Labor, sondern auch unter Produktionsbedingungen gute Ergebnisse zu erzielen sind. Es wurde ein Versuch durchgeführt, wie er in Sammelkampagnen an beliebigen Orten ablaufen kann.

### B) Versuchsdurchführung

Frischer Harn ausreichender Qualität wurde in einem Gestüt gesammelt. Bei drei Pferden und 15 bis 20 1 Harn/Tag fielen an 10 Tagen insgesamt etwa 150 bis 200 1 an. Zur Adsorption wurde deshalb eine 5 1 Adsorbersäule gewählt. Die tägliche Beladung mit Harn, außer am Wochenende, wurde im Versuch nach folgendem Plan durchgeführt:

| | |
|---|---|
| Start der Harnsammlung am Wochenende | |
| Montag : | Aufgabe des Harns vom Sonnabend, Sonntag und Montag |
| Dienstag bis Freitag: | Aufgabe des Harns vom jeweiligen Tag |
| Montag : | Aufgabe des Harns vom Sonnabend, Sonntag und Montag |
| Dienstag : | Eluierung |

Die Harnaufgabe mit einer Beladegeschwindigkeit von ca. 4,5 Bettvolumen/h erfolgte direkt auf die Säule, lediglich eine Baumwollkerze mit einer Trenngrenze von 20 µm diente als Vorfilter. Zwischen den einzelnen Aufgaben wurde zur Erschwerung der Bedingungen keine Wasserspülung vorgenommen. In Vorversuchen konnten bis zu 450 1 Harn ohne Druckverlust über eine Baumwollkerze gegeben werden. In diesem Praxistest erwies sich die Filtration aufgrund eines unerwartet hohen Gehaltes an Calciumcarbonat-Sediment und/oder an Muccinen allerdings als schwierig. Nach jeweils 40 bis 50 1 trat eine Verblockung mit einem

Nach jeweils 40 bis 50 1 trat eine Verblockung mit einem Druckanstieg bis ca. 4 bar auf, die einen Filteraustausch erforderlich machten. Je nach Gehalt an Calciumcarbonat und/oder Muccinen empfiehlt sich daher gegebenenfalls der Zusatz von Filterhilfsmitteln.

Obwohl die Kerze Sedimentanteile > 20 µm passieren ließ, war ein Zusetzen der Adsorbersäule nicht gegeben.

### C) Ergebnisse

### Hormongehalte

Die Ergebnisse dieses Praxistests sind in Tabelle IV wiedergegeben.

Die Estron- und Equilingehalte des Rohurins waren mit 123 mg/l bzw. 74 mg/l erfreulich hoch und entsprechen einem Harn mittlerer bis guter Qualität. Unerwartet groß waren jedoch die Kresolwerte mit 531 mg/l im frischen Urin, die bisher nicht beobachtet wurden. Hier spielt die Fütterungsfrage hinein und bedarf der Berücksichtigung. Obwohl grundsätzlich z.B. höhere Kresolgehalte beherrscht werden, können sie im Einzelfall doch Schwierigkeiten verursachen. Es kann deshalb erforderlich sein den alkalischen Waschprozeß bei der Aufarbeitung zu verlängern, um die Kresolwerte im ethanolischen Eluat zu minimieren. Ansonsten verliefen die Adsorption und Desorption wie gewohnt. Im Eluat war ein gut ausgeprägter Verlauf für Estron und Equilin gegeben. In den Eluaten 2 und 3 fanden sich mehr als 95 % an Estron/Equilin wieder. Bezogen auf den TS-Gehalt beträgt der Gesamthormongehalt ca. 30 Gew.-%. Damit wurde die in den Versuch gesetzte Erwartung erfüllt und unter schwierigen Versuchsbedingungen eine hervorragende Anreicherung auf einen Hormongehalt/TS von > 15 Gew.-% erreicht.

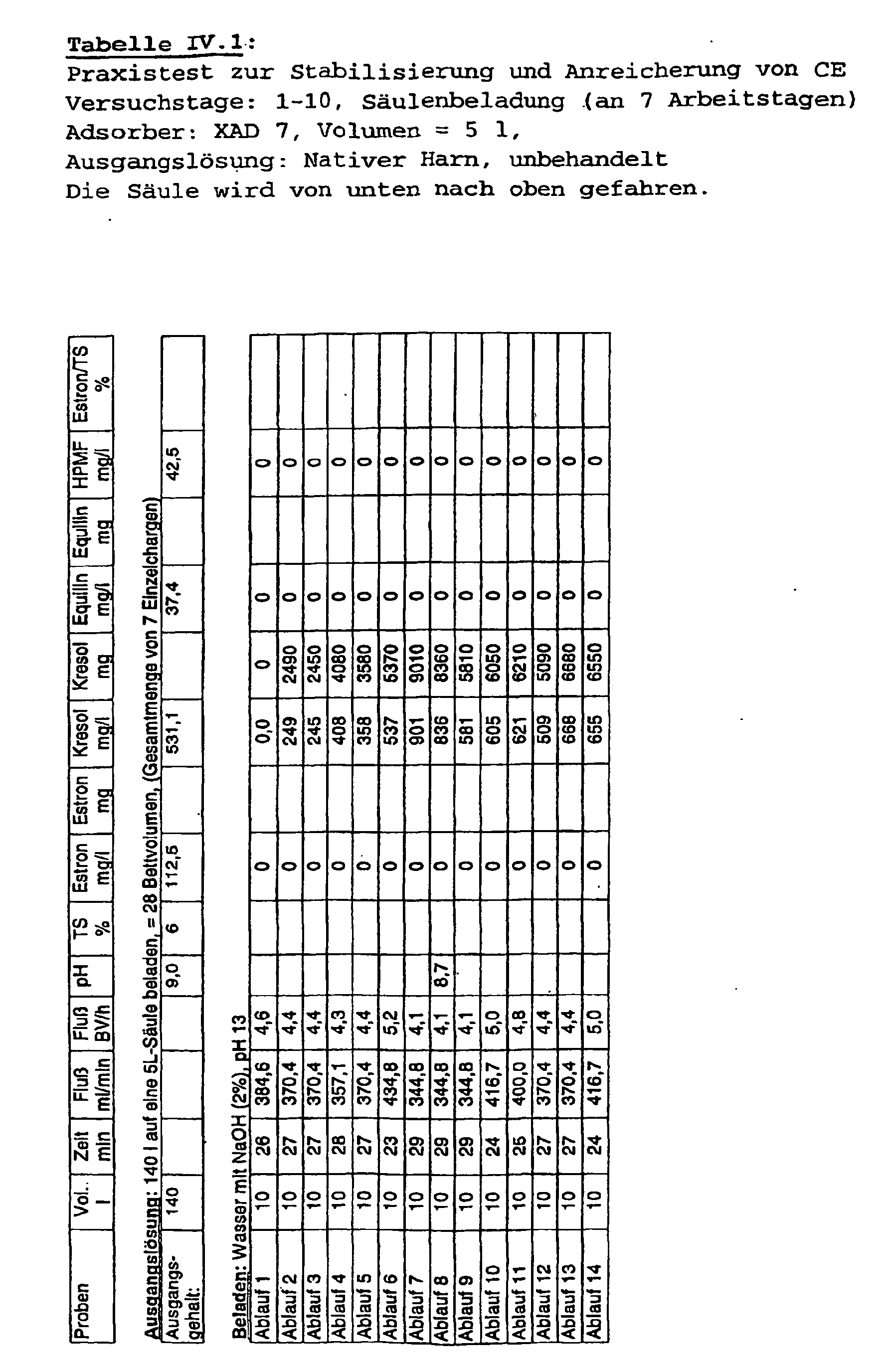

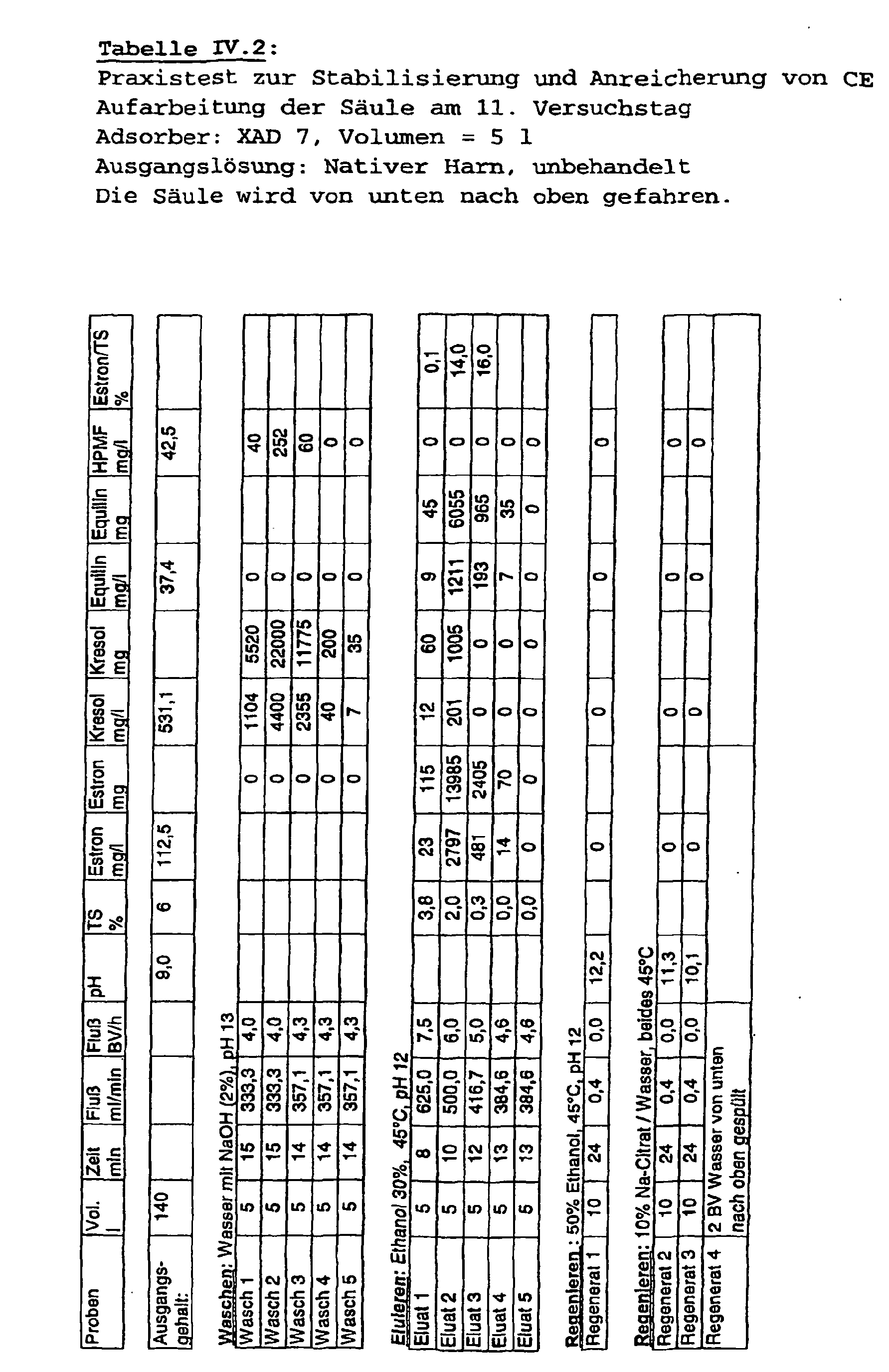

### Keimzahlen

Zusätzlich zur Harnadsorption wurde ein möglicher bakterieller Keimbewuchs der Säule untersucht. Bei den vorliegenden Bedingungen:
1. Aufgabe von kontaminierten Rohurin (durch die Baumwollkerzenfiltration werden keine Keime abgetrennt)
2. Keine Spülung nach der täglichen Aufgabe
3. Versuchsdurchführung bei Raumtemperatur
4. Versuchsdauer von 10 Tagen
war eine Keimbesiedlung der Säule und ein sich daran anschließendes Keimwachstum nicht auszuschließen. Eine Bestimmung der Gesamtkeimzahl bei verschiedenen Adsorptionsschritten ergab folgendes Ergebnis:

| Probe | Gesamtkeimzahl / ml |
|---|---|
| Rohurin | 1,1 x 10⁸ |
| Säulen-Waschwasser nach 10 Tagen | 1,1 x 10⁸ |
| Basisches Waschwasser | < 1 |
| Haupteluat | 1,5 |

### D) Zusammenfassung der Ergebnisse

Über einen Versuchszeitraum von 10 Tagen wurde frischer Harn täglich auf eine 5 1 Adsorbersäule gegeben. Das gesamte Harnvolumen betrug 140 1 mit einem mittleren Estron- bzw. Equilingehalt von 123 mg/l bzw. 74 mg/l.

Auf eine Säulenspülung mit Wasser nach der täglichen Harnadsorption wurde bewußt verzichtet. In den beiden sehr ausgeprägt anfallenden Haupteluaten fanden sich ca. 93 % an Estron bzw. Equilin wieder. Der Gesamthormongehalt des Eluates bezogen auf TS betrug ca. 30 Gew.-%. Ein weiteres überraschendes Ergebnis bezieht sich auf eine denkbare Säulenverkeimung. Hier war trotz Aufgabe von hochverkeimten Rohurin ein Bewuchs der Säule selbst nach 10 Tagen nicht erkennbar. Das anfallende Eluat war mit 1,5 Keimen/ml fast keimfrei. Unter schwierigen Bedingungen wurde damit im größerem Maßstab nachgewiesen, daß bei ausreichendem Hormongehalt im Rohurin eine Stabilisierung und Anreicherung auf einen Hormongehalt/TS von größer 15 % mit einer Adsorbersäule in guter Qualität erfüllt werden kann.

### Beispiel 6:

### Filtrationsversuche mit Filterkerzen

In einem weiteren Praxistest wurden Filtrationsversuche mit Filterkerzen durchgeführt, deren Ergebnisse wie folgt zusammengefaßt werden können:

Verwendetes Material: frischer Urin (Herkunft aus einem deutschen Gestüt)

Der zum Teil aufgerührte, in Fässern gesammelte Urin trächtiger Stuten wurde aus den Fässern mittels einer Schlauchpumpe über einen 5µ Beutel (Vorfilter) in einen Container umgepumpt. Im Container verblieb ein Sediment von etwa 0,5 Gew.-%. Eine schematische Darstellung der Verfahrensweise zur Filtration von Rohurin ist in Fig. 2 wiedergegeben.

| 1. Versuch: | |
|---|---|
| Beutel | 5µ |
| Filterkerzen | 5µ, 3µ, 1µ in Serie geschaltet |
| Absolutkerzen | 2 x 1,2µ parallel geschaltet |
| Säulenbeladung | 95 1 Filtrat |
| Sediment Filtrat | 0,000 % |

| 2. Versuch: | |
|---|---|
| Beutel | 5µ |
| Filterkerzen | 5µ, 3µ, 1µ in Serie geschaltet |
| Absolutkerze | 2 x 3µ parallel geschaltet |
| Säulenbeladung | 142 1 Filtrat |
| Sediment Filtrat | 0,001 % |

| 3. Versuch: | |
|---|---|
| Beutel | 5µ |
| Filterkerzen | 3µ, 1µ in Serie geschaltet |
| Absolutkerze | 2 x 1µ parallel geschaltet |
| Säulenbeladung | 160 1 Filtrat |
| Sediment Filtrat | 0,000 % |

Es wurde je Versuch eine Probe nach der Absolutkerze (Membranfilter) genommen. Alle 100 l wurde eine Probe aus dem Ablauf, nach der Säule, genommmen.

### Gesamte Säulenbeladung: 397 1 Filtrat

Der Trockensubstanzgehalt nach Aufarbeitung (TS)betrug 5,9 Gew.-%.

## Patentansprüche

1. Verfahren zur praxisgerechten dezentralen Anreicherung und Stabilisierung von Gemischen konjugierter Oestrogene (CE) aus dem Harn trächtiger Stuten (PMU), welches sich **dadurch** auszeichnet, daß
- das im Harn trächtiger Stuten (PMU) enthaltende natürliche Gemisch konjugierter Oestrogene (CE) an einem festen Träger (= Adsorbens) angereichert und stabilisiert wird, indem
- eine vorgegebene maximale Gesamtmenge einer gesammelten, gegebenenfalls zuvor grob mechanisch vorgereinigte Harn-flüssigkeit aus einem Vorratsgefäß kontinuierlich oder in diskreten Teilmengen mit vorgegebener Strömungsgeschwindigkeit durch eine aufrecht angeordnete Kartusche gepumpt wird und die abfließende Harnflüssigkeit verworfen wird, wobei
- die Kartusche mit einem von Flüssigkeit umgebenen, zur Adsorption einer vordefinierten Menge des in der Harnflüssigkeit enthaltenen Gemisches konjugierter Oestrogene (CE) geeigneten Adsorbens gefüllt ist, und wobei
- die als Endpunktskontrolle für die Beladung des Adsorbens vorgegebene, maximale Gesamtmenge der durch die Kartusche gepumpten Harnflüssigkeit auf die maximale Beladungskapazität des Adsorbens für die in der Harnflüssigkeit enthaltenen konjugierten Oestrogene (CE) abgestimmt ist.

2. Verfahren nach Anspruch 1, **dadurch gekennzeichnet, daß** die Kartuschen-Innenabmessungen für das Adsorbensbett so bemessen sind, daß die Kartusche 30 bis 50 1 Adsorbens aufnehmen kann und der PMU vom Einlaß bis zum Auslaß eine Wegstrecke von 80 bis 120 cm zurücklegen muß.

3. Verfahren nach Anspruch 1, **dadurch gekennzeichnet, daß** die im Verfahren verwendete Kartusche aus schlagfestem Laborglas, Kunststoff und/oder Metall besteht.

4. Verfahren nach Anspruch 1, **dadurch gekennzeichnet, daß** als Pumpe eine Monopumpe, Schlauchpumpe oder Membranpumpe eingesetzt wird.

5. Verfahren nach Anspruch 1, **dadurch gekennzeichnet, daß** als Strömungsmesser ein Rotameter mit Schwimmkörper, ein Drehflügelströmungsmesser oder ein induktiver Strömungsmesser eingesetzt wird.

6. Verfahren nach Anspruch 1, **dadurch gekennzeichnet, daß** zur Messung der über die Kartusche gepumpten Menge an Harnflüssigkeit ein Durchflußmesser, vorzugsweise eine Wasseruhr oder ein induktiver Durchflußmesser, eingesetzt wird.

7. Verfahren nach Anspruch 1, **dadurch gekennzeichnet, daß** die Strömungsgeschwindigkeit (= Beladegeschwindigkeit) im Bereich von 3 bis 10 Adsorber-Bettvolumen/h, vorzugsweise 4 bis 6 Adsorber-Bettvolumen/h, liegt.

8. Verfahren nach Anspruch 1, **dadurch gekennzeichnet, daß** als Adsorbens polymere Adsorberharze, Kieselgel oder RP-Kieselgel, insbesondere polymere Adsorberharze, vorzugsweise semipolare polymere Adsorberharze, eingesetzt werden.

9. Verfahren nach Anspruch 1, **dadurch gekennzeichnet, daß** die maximale Gesamtmenge an Harnflüssigkeit 20 bis 60 Adsorber-Bettvolumen, vorzugsweise 30 bis 40 Adsorber-Bettvolumen beträgt

10. Verfahren nach Anspruch 1, **dadurch gekennzeichnet, daß** die Kartusche nach der Beladung des Adsorbens mit einer in etwa der Gesamtkapazität des Adsorbens entsprechenden Menge PMU oder auch bereits vor Erreichen der Gesamtbeladungskapazität nach beliebigen einzelnen Teilbeladungsschritten mit Wasser und/oder einer anderen geeigneten wäßrigen Waschlösung gespült wird.

11. Verfahren nach Anspruch 1, **dadurch gekennzeichnet, daß** die Kartusche nach der Beladung des Adsorbers gewünschtenfalls zunächst mit Wasser und/oder einer anderen geeigneten wäßrigen Waschlösung und abschließend zur weiteren Stabilisierung mit einer konservierenden Lösung, z.B. mit einer Konservierungsmittel enthaltenden Lösung, einer pH-eingestellten wäßrigen Lösung und/oder einer wäßrigen Salzlösung gespült wird.

12. Verfahren nach Anspruch 1, **dadurch gekennzeichnet, daß** die Harnflüssigkeit aus dem Vorratsbehälter zunächst erst über einen oder mehrere Vorfilter gepumpt wird, vorzugsweise wenigstens über einen Tiefenfilter oder einen Anschwemmfilter, bevor die Harnflüssigkeit über die Kartusche gepumpt wird.

13. Verfahren nach Anspruch 12, **dadurch gekennzeichnet, daß** der Vorfilter ein Tiefenfilter mit Sandbett oder eine Filterpatrone, ein Plattenfilter, ein Kerzenfilter, Filterbeutel oder Filtrationsrohr ist.

14. Verfahren nach Anspruch 12 oder 13, **dadurch gekennzeichnet, daß** man der Harnflüssigkeit vor der Filtration Filtrationshilfsmittel zusetzt.

15. Vorrichtung zur praxisgerechten dezentralen Anreicherung und Stabilisierung von Gemischen konjugierter Oestrogene (CE) aus dem Harn trächtiger Stuten (PMU) auf einen festen Träger (Adsorbens), umfassend
- eine aufrecht angeordnete Kartusche, die mit einem (ständig) von Flüssigkeit umgebenen, zur Adsorption einer vordefinierten Menge des in der Harnflüssigkeit trächtiger Stuten enthaltenen Gemisches konjugierter Oestrogene (CE) geeigneten Adsorbens gefüllt ist und wobei die Kartusche entweder a) einen fußseitig angeordneten Flüssigkeitseinlaß und einen kopfseitig angeordneten Flüssigkeitsauslaß oder b) einen kopfseitig angeordneten Flüssigkeitseinlaß und einen fußseitig angeordneten Flüssigkeitsauslaß aufweist,
- sowie vor der Kartusche in der angegebenen Reihenfolge angeordnet und durch Schlauchleitungen miteinander verbunden
- eine Pumpe, einen Strömungsmesser und einen Durchflußsmesser.

16. Vorrichtung nach Anspruch 15, **dadurch gekennzeichnet, daß** zwischen Pumpe und Strömungsmesser zusätzlich wenigstens ein Vorfilter, vorzugsweise ein Tiefenfilter oder ein Anschwemmfilter, zwischengeschaltet ist.

17. Vorrichtung nach Anspruch 16, **dadurch gekennzeichnet, daß** der Vorfilter ein Modul aus zwei parallel geschalteten Vorfiltern ist, die im Wechsel einzeln betrieben und auch bei laufendem Betrieb der Vorrichtung ausgetauscht werden können.

18. Vorrichtung nach Anspruch 16 oder 17, **dadurch gekennzeichnet, daß** zur Kontrolle der ordnungsgemäßen Funktion des Vorfilters ein Manometer zwischen Pumpe und Vorfilter sowie ein Manometer im Bereich nach dem Vorfilter, vorzugsweise hinter dem Strömungsmesser angeordnet, zwischengeschaltet ist.

19. Vorrichtung nach Anspruch 15, **dadurch gekennzeichnet, daß** die Kartusche Innenabmessungen für das Adsorbensbett mit einer Innenhöhe etwa im Bereich von 80 bis 120 cm und einem Innendurchmesser etwa im Bereich von 10 bis 25 cm aufweist.

20. Vorrichtung nach Anspruch 15, **dadurch gekennzeichnet, daß** die von der Vorrichtung umfaßte Kartusche aus schlagfestem Laborglas, Kunststoff und/oder Metall besteht.

21. Vorrichtung nach Anspruch 15, **dadurch gekennzeichnet, daß** die von der Vorrichtung umfaßte Pumpe eine Monopumpe, Schlauchpumpe oder Membranpumpe ist.

22. Vorrichtung nach Anspruch 15, **dadurch gekennzeichnet, daß** der von der Vorrichtung umfaßte Strömungsmesser ein Rotameter mit Schwimmkörper, ein Drehflügelströmungsmesser oder ein induktiver Strömungsmesser ist.

23. Vorrichtung nach Anspruch 15, **dadurch gekennzeichnet, daß** der von der Vorrichtung umfaßte, zur Messung der über die Kartusche gepumpten Menge an Harnflüssigkeit dienende Durchflußmesser eine Wasseruhr oder ein induktiver Durchflußmesser ist.

## Claims

1. A method for the concentration and stabilisation, which is practicable and decentralised, of mixtures of conjugated oestrogens (CE) from pregnant mares' urine (PMU), which is distinguished in that
- the natural mixture of conjugated oestrogens (CE) contained in the pregnant mares' urine (PMU) is concentrated and stabilised on a solid support (= adsorbent), in that
- a predetermined maximum total quantity of a collected urine, which has optionally been pre-purified coarsely mechanically beforehand, is pumped from a storage vessel continuously or in discrete partial quantities at a predetermined flow rate through an upright cartridge and the discharged urine is discarded, wherein
- the cartridge is filled with an adsorbent surrounded by liquid and suitable for the adsorption of a predefined amount of the mixture of conjugated oestrogens (CE) contained in the urine, and wherein
- the maximum total quantity of the urine pumped through the cartridge preset as end point control for the loading of the adsorbent is matched to the maximum loading capacity of the adsorbent for the conjugated oestrogens (CE) contained in the urine.

2. A method according to Claim 1, **characterised in that** the internal dimensions of the cartridge for the adsorbent bed are calculated such that the cartridge can hold 30 to 50 1 adsorbent and the PMU has to cover a distance of 80 to 120 cm from the inlet to the outlet.

3. A method according to Claim 1, **characterised in that** the cartridge used in the method is made of impact-resistant laboratory glass, plastics and/or metal.

4. A method according to Claim 1, **characterised in that** a monopump, hose pump or diaphragm pump is used as the pump.

5. A method according to Claim 1, **characterised in that** a rotameter with float, a rotary-vane flow meter or an inductive flow meter is used as flow meter.

6. A method according to Claim 1, **characterised in that** a throughflow meter, preferably a water meter or an inductive throughflow meter, is used for measuring the quantity of urine pumped through the cartridge.

7. A method according to Claim 1, **characterised in that** the flow rate (= loading rate) lies in the range of 3 to 10 bed volumes adsorber/h, preferably 4 to 6 bed volumes adsorber/h.

8. A method according to Claim 1, **characterised in that** polymeric adsorption resins, silica gel or RP-silica gel, in particular polymeric adsorption resins, preferably semipolar polymeric adsorption resins, are used as adsorbent.

9. A method according to Claim 1, **characterised in that** the maximum total quantity of urine is 20 to 60 bed volumes adsorber, preferably 30 to 40 bed volumes adsorber.

10. A method according to Claim 1, **characterised in that** the cartridge after the loading of the adsorbent with an amount of PMU corresponding approximately to the total capacity of the adsorbent or alternatively even before reaching the total loading capacity after any individual partial loading steps is rinsed with water and/or another suitable aqueous washing solution.

11. A method according to Claim 1, **characterised in that** the cartridge after the loading of the adsorber if desired is rinsed initially with water and/or another suitable aqueous washing solution and finally for further stabilisation with a preserving solution, e.g. with a preservative-containing solution, a pH-adjusted aqueous solution and/or an aqueous salt solution.

12. A method according to Claim 1, **characterised in that** the urine from the storage container is first pumped through one or more prefilters, preferably at least through a depth filter or a precoat filter, before the urine is pumped through the cartridge.

13. A method according to Claim 12, **characterised in that** the prefilter is a depth filter with sand bed or a filter cartridge, a plate filter, a cartridge filter, filter bag or filtration tube.

14. A method according to Claim 12 or 13, **characterised in that** filtration aids are added to the urine before the filtration.

15. A device for the concentration and stabilisation, which is practicable and decentralised, of mixtures of conjugated oestrogens (CE) from pregnant mares' urine (PMU), on a solid support (adsorbent), comprising
- an upright cartridge, which is filled with an adsorbent (constantly) surrounded by liquid and suitable for the adsorption of a predefined amount of the mixture of conjugated oestrogens (CE) contained in pregnant mares' urine, and wherein the cartridge either a) has a liquid inlet located at the bottom and a liquid outlet located at the top or b) has a liquid inlet located at the top and a liquid outlet located at the bottom,
- and also located before the cartridge in the given sequence and joined together by hoses
- a pump, a flow meter and a throughflow meter.

16. A device according to Claim 15, **characterised in that** additionally at least one prefilter, for example a depth filter or a precoat filter, is interposed between the pump and flow meter.

17. A device according to Claim 16, **characterised in that** the prefilter is a module consisting of two prefilters connected in parallel, which can be operated individually alternately and also can be replaced while operation of the device continues.

18. A device according to Claim 16 or 17, **characterised in that** for monitoring the proper functioning of the prefilter a manometer is interposed between the pump and prefilter, and a manometer in the region after the prefilter, preferably after the flow meter.

19. A device according to Claim 15, **characterised in that** the cartridge has internal dimensions for the adsorbent bed with an internal height approximately in the range of 80 to 120 cm and an internal diameter approximately in the range of 10 to 25 cm.

20. A device according to Claim 15, **characterised in that** the cartridge which the device surrounds is made of impact-resistant laboratory glass, plastics and/or metal.

21. A device according to Claim 15, **characterised in that** the pump which the device surrounds is a monopump, hose pump or diaphragm pump.

22. A device according to Claim 15, **characterised in that** the flow meter which the device surrounds is a rotameter with float, a rotary-vane flow meter or an inductive flow meter.

23. A device according to Claim 15, **characterised in that** the throughflow meter which the device surrounds which is used for measuring the quantity of urine pumped through the cartridge is a water meter or an inductive throughflow meter.

## Revendications

1. Procédé d'enrichissement et de stabilisation décentralisés, conformes à la pratique, de mélanges d'oestrogènes conjugués (CE) provenant de l'urine de juments gravides (PMU), qui est **caractérisé en ce que**
- on enrichit et stabilise sur un support solide (adsorbant) le mélange naturel, contenu dans l'urine de juments gravides (PMU), d'oestrogènes conjugués (CE),
- on pompe à partir d'un réservoir, en continu ou en quantités partielles séparées, à une vitesse d'écoulement prédéfinie, et à travers une cartouche disposée verticalement, une quantité totale maximale prédéterminée d'un liquide urinaire combiné, qui a été éventuellement soumis au préalable à une pré-épuration mécanique, et on évacue le liquide urinaire qui s'écoule, moyennant quoi
- on remplit la cartouche avec un adsorbant entouré de liquide, convenant à l'adsorption d'une quantité prédéfinie du mélange, contenu dans le liquide urinaire, d'oestrogènes conjugués (CE), et
- on adapte la quantité totale maximale, prédéfinie en tant que contrôle du point final pour la charge de l'adsorbant, du liquide urinaire pompé à travers la cartouche, à la capacité maximale de charge de l'adsorbant pour les oestrogènes conjugués (CE) contenus dans le liquide urinaire.

2. Procédé selon la revendication 1, **caractérisé en ce que** les dimensions intérieures de la cartouche, destinée au lit d'adsorbant, sont définies de telle sorte que la cartouche puisse recevoir 30 à 50 1 d'adsorbant, et que le PMU, du point d'entrée au point de sortie, doive parcourir une distance de 80 à 120 cm.

3. Procédé selon la revendication 1, **caractérisé en ce que** la cartouche utilisée dans le procédé est constituée d'un verre de laboratoire résistant au choc, d'une matière plastique et/ou d'un métal.

4. Procédé selon la revendication 1, **caractérisé en ce qu'**on utilise en tant que pompe une monopompe, une pompe à tube flexible ou une pompe à membrane.

5. Procédé selon la revendication 1, **caractérisé en ce qu'**on utilise en tant qu'appareil pour mesurer l'écoulement un rotamètre comportant un flotteur, un moulinet à organe rotatif, ou un appareil à induction.

6. Procédé selon la revendication 1, **caractérisé en ce que**, pour mesurer la quantité de liquide urinaire pompée à travers la cartouche, on utilise un débitmètre, de préférence un compteur d'eau ou un débitmètre à induction.

7. Procédé selon la revendication 1, **caractérisé en ce que** la vitesse d'écoulement (= vitesse de charge) est comprise dans la plage de 3 à 10 volumes de lit d'adsorbant/h, de préférence de 4 à 6 volumes de lit d'adsorbant/h.

8. Procédé selon la revendication 1, **caractérisé en ce qu'**on utilise en tant qu'adsorbant des résines adsorbantes polymères, du gel de silice ou du gel de silice RP, en particulier des résines adsorbantes polymères, de préférence des résines adsorbantes polymères semi-polaires.

9. Procédé selon la revendication 1, **caractérisé en ce que** la quantité totale maximale de liquide urinaire est de 20 à 60 volumes de lit d'adsorbant, de préférence de 30 à 40 volumes de lit d'adsorbant.

10. Procédé selon la revendication 1, **caractérisé en ce que** la cartouche, après la charge de l'adsorbant, est rincée avec une quantité de PMU correspondant approximativement à la capacité totale de l'adsorbant, ou aussi, même avant d'atteindre la capacité totale de charge après des étapes individuelles quelconques de charge partielle, avec de l'eau et/ou avec une autre solution aqueuse de lavage appropriée.

11. Procédé selon la revendication 1, **caractérisé en ce que** la cartouche, après la charge de l'adsorbant, est si on le souhaite, rincée d'abord avec de l'eau et/ou avec une autre solution aqueuse appropriée de lavage, et finalement, pour assurer une stabilisation plus poussée, avec une solution à effet conservateur, par exemple avec une solution contenant un conservateur, avec une solution aqueuse dont on a ajusté le pH, et/ou avec une solution saline aqueuse.

12. Procédé selon la revendication 1, **caractérisé en ce que** le liquide urinaire est pompé, à partir du réservoir, d'abord seulement sur un ou plusieurs préfiltres, de préférence sur au moins un filtre à lit profond ou un filtre à couches, avant que le liquide urinaire soit pompé à travers la cartouche.

13. Procédé selon la revendication 12, **caractérisé en ce que** le préfiltre est un filtre à lit profond comportant un lit de sable, ou une cartouche filtrante, un filtre à plaques, un filtre à bougie, une poche filtrante ou un tube filtrant.

14. Procédé selon la revendication 12 ou 13, **caractérisé en ce que**, avant la filtration, on ajoute des auxiliaires de filtration au liquide urinaire.

15. Dispositif pour l'enrichissement et la stabilisation décentralisés, conformes à la pratique, de mélanges d'oestrogènes conjugués (CE), provenant de l'urine de juments gravides (PMU) sur un support solide (adsorbant), comprenant
- une cartouche disposée verticalement, qui est remplie d'un adsorbant entouré (en permanence) de liquide, convenant à l'adsorption d'une quantité prédéfinie du mélange, contenu dans le liquide urinaire de juments gravides, d'oestrogènes conjugués (CE), la cartouche comportant soit a) un orifice d'entrée de liquide, disposé côté pied, et un orifice de sortie de liquide, disposé côté tête, soit b) un orifice d'entrée de liquide disposé côté tête et un dispositif de sortie de liquide disposé côté pied,
- ainsi que, disposés en amont de la cartouche dans l'ordre indiqué et communiquant les uns avec les autres par des tuyaux,
- une pompe, un moulinet et un débitmètre.

16. Dispositif selon la revendication 15, **caractérisé en ce qu'**entre la pompe et le moulinet, est intercalé en outre au moins un préfiltre, de préférence un filtre à lit profond ou un filtre à couches.

17. Dispositif selon la revendication 16, **caractérisé en ce que** le préfiltre est un module de deux préfiltres montés en parallèle, qui sont exploités en alternance à titre individuel, et qui aussi peuvent être échangés en cours d'exploitation continue du dispositif.

18. Dispositif selon la revendication 16 ou 17, **caractérisé en ce que**, pour le contrôle du bon fonctionnement du préfiltre, est intercalé un manomètre entre la pompe et le préfiltre, ainsi qu'un manomètre dans la zone située en aval du préfiltre, de préférence en aval du moulinet.

19. Dispositif selon la revendication 15, **caractérisé en ce que** la cartouche présente des dimensions intérieures, pour le lit d'adsorbant, correspondant à une hauteur intérieure comprise approximativement dans la plage de 80 à 120 cm, et un diamètre intérieur compris approximativement dans la plage de 10 à 25 cm.

20. Dispositif selon la revendication 15, **caractérisé en ce que** la cartouche incorporée dans le dispositif est constituée d'un verre de laboratoire résistant au choc, d'une matière plastique et/ou d'un métal.

21. Dispositif selon la revendication 15, **caractérisé en ce que** la pompe incorporée dans le dispositif est une monopompe, une pompe à tube souple ou une pompe à membrane.

22. Dispositif selon la revendication 15, **caractérisé en ce que** l'appareil inclus dans le dispositif pour mesurer l'écoulement est un rotamètre à plongeur, un moulinet à élément rotatif ou un appareil à induction.

23. Dispositif selon la revendication 15, **caractérisé en ce que** le débitmètre inclus dans le dispositif, pour mesurer la quantité de liquide urinaire pompé à travers la cartouche, est un compteur d'eau ou un débitmètre à induction.
